# EUROPEAN PATENT APPLICATION

(11) **EP 3 854 869 A1**
(43) Date of publication of application: **28.07.2021**
(21) Application number: 19863748.0
(22) Date of filing: 18.09.2019
(51) Int. Cl.: C12N 5/071, A61K 35/12, A61P 3/10, A61P 5/50

(54) **INSULIN-PRODUCING CELLS**

(30) Priority: 19.09.2018 JP 2018175465
(71) Applicant: Takeda Pharmaceutical Company Limited, Osaka-shi, Osaka 541-0045 (JP); Kyoto University, Kyoto-shi, Kyoto 606-8501 (JP)
(72) Inventor: ITO, Ryo, Fujisawa-shi, Kanagawa 251-0012 (JP); YAMAZOE, Noriko, Fujisawa-shi, Kanagawa 251-0012 (JP); HIYOSHI, Hideyuki, Fujisawa-shi, Kanagawa 251-0012 (JP); MOCHIDA, Taisuke, Fujisawa-shi, Kanagawa 251-0012 (JP); UENO, Hikaru, Fujisawa-shi, Kanagawa 251-0012 (JP); SAKUMA, Kensuke, Fujisawa-shi, Kanagawa 251-0012 (JP); YAMAURA, Junji, Fujisawa-shi, Kanagawa 251-0012 (JP); MATSUMOTO, Hirokazu, Fujisawa-shi, Kanagawa 251-0012 (JP); TOYODA, Taro, Kyoto-shi, Kyoto 606-8501 (JP); KONAGAYA, Shuhei, Kyoto-shi, Kyoto 606-8501 (JP)
(74) Representative: Jones, Nicholas Andrew
(86) International application number: PCT/JP2019/037725
(87) International publication number: WO 2020/059892

(57) **Abstract**

The purpose of the present invention is to provide a novel method for making it possible to efficiently induce/produce endocrine cells from pluripotent stem cells. Insulin-producing cells including cells that are insulin-positive and NKX6.1-positive cells in a ratio of at least 30% and cells that are insulin-positive and NKX6.1-negative in a ratio of more than 15%.

## Description

### Technical Field

The present invention relates to insulin-producing cells that enable efficient induction/production of endocrine cells that secrete hormones such as pancreatic β cells and pancreatic α cells, a method for producing the insulin-producing cells, and applications of the insulin-producing cells.

### Background Art

Research is underway to induce the differentiation of pluripotent stem cells such as induced pluripotent cells and embryonic-stem cells (ES cells) into endocrine cells that secrete hormones such as pancreatic β cells and pancreatic α cells and to apply the obtained cells to the treatment of diabetes mellitus. It is known that cells having different features depending on the stages of differentiation appear when the differentiation of pluripotent stem cells is induced (WO2009/012428, WO2016/021734). For example, the stages of differentiation can be broadly classified into pluripotent stem cells, definitive endoderm cells, primitive gut tube cells, posterior foregut cells, pancreatic progenitor cells, endocrine progenitor cells, insulin-producing cells, and pancreatic β cells in order from relatively undifferentiated to differentiated forms.

Previously, approaches to induce the differentiation of pluripotent stem cells into endocrine cells including insulin-positive cells have been developed and reported. For example, Non Patent Literatures 1 and 2 describe methods for producing insulin-producing cells formed by inducing the differentiation of pluripotent stem cells and comprising insulin-positive and NKX6.1-positive cells at a proportion of 30% or more and insulin-positive and NKX6.1-negative cells at a proportion of 15% or less.

In addition, approaches to produce endocrine cells by inducing the differentiation of pluripotent stem cells into pancreatic progenitor cells and transplanting the pancreatic progenitor cells in the living body and maturing the pancreatic progenitor cells have been reported. For example, Non Patent Literature 3 describes a method for producing endocrine cells comprising insulin-positive cells and glucagon-positive cells by transplanting pancreatic progenitor cells formed by inducing the differentiation of pluripotent stem cells into a mouse and maturing the pancreatic progenitor cells.

However, the proportion of endocrine cells produced by any of the above methods is never sufficiently high, and a method for efficiently inducing/producing endocrine cells from pluripotent stem cells is desired in the art still now.

### Citation List

### Patent Literature

Patent Literature 1: WO2009/012428
Patent Literature 2: WO2016/021734

### Non Patent Literature

Non Patent Literature 1: Rezania A. et al, Nat Biotechnol. 2014; 32: 1121-33.
Non Patent Literature 2: Felicia W. Pagliuca et al, Cell. 2014 October 9; 159(2): 428-439.
Non Patent Literature 3: Robert T et al, Stem Cell Reports. 2018 Mar 13; 10(3): 739-750.

### Summary of Invention

### Technical Problem

An object of the present invention is to provide a novel approach that enables efficient induction/production of pancreatic islet-like cells comprising specific proportions of insulin-positive cells and glucagon-positive cells from pluripotent stem cells.

### Solution to Problem

While previous studies mainly focused on how to increase the proportion of insulin-positive and NKX6.1-positive cells and mature the cells, the present inventors have conducted diligent studies to attain the object and consequently found that inclusion of a specific proportion of cells other than those mentioned above enables efficient induction/production of pancreatic islet-like cells with high purity in the living body. Induced is the differentiation into insulin-producing cells with less maturation than insulin-producing cells produced through a conventional approach, specifically, insulin-producing cells comprising many cells not expressing a maturation marker such as MafA and being to become glucagon-producing cells. Cells with a low degree of maturation may give cells with low purity after transplantation; however, the present inventors succeeded in maturing into pancreatic islet-like cells with high purity kept in the living body by combining an approach to reduce Ki67-positive cells, which have proliferative capacity, to the utmost limit.

The present invention can efficiently induce pancreatic islet-like cells with high purity within the shortest manufacturing period, and hence provides the most suitable approach to achieve cell therapy for the pancreatic islet.

The present invention is based on these novel findings and encompasses the following inventions.
[1] Insulin-producing cells (cell population) comprising: insulin-positive and NKX6.1-positive cells at a proportion of 30% or more; and insulin-positive and NKX6.1-negative cells at a proportion of more than 15%.
[2] The insulin-producing cells (cell population) according to [1], wherein an expression level of a MafA gene or a gene product thereof is lower than an expression level of a MafA gene or a gene product thereof in a pancreatic islet.
[3] The insulin-producing cells (cell population) according to [1] or [2], comprising Ki67-positive cells at a proportion of less than 3%.
   [3-1] The insulin-producing cells (cell population) according to [1] or [2], comprising Ki67-positive cells at a proportion of less than 1%.
[4] The insulin-producing cells (cell population) according to any of [1] to [3-1], comprising glucagon-positive and insulin-negative cells at a proportion of less than 3%.
[5] The insulin-producing cells (cell population) according to any of [1] to [4], wherein the insulin-producing cells exhibit glucose-stimulated insulin secretion (GSIS) response.
[6] The insulin-producing cells (cell population) according to any of [1] to [5], comprising chromogranin A-positive cells at a proportion of more than 45%.
   [6-1] The insulin-producing cells (cell population) according to any of [1] to [5], comprising chromogranin A-positive cells at a proportion of more than 80%.
   [6-2] The insulin-producing cells (cell population) according to any of [1] to [5], comprising chromogranin A-positive cells at a proportion of more than 85%.
   [6-3] The insulin-producing cells (cell population) according to any of [1] to [5], comprising chromogranin A-positive cells at a proportion of more than 90%.
   [6-4] The insulin-producing cells (cell population) according to any of [1] to [5], comprising chromogranin A-positive cells at a proportion of more than 93%.
[7] The insulin-producing cells (cell population) according to any of [1] to [6-4], comprising alkaline phosphatase-positive pluripotent stem cells at a proportion of less than 0.01%.
   [7-1] The insulin-producing cells (cell population) according to any of [1] to [7], wherein the insulin-producing cells differentiate into pancreatic islet-like cells in a living body.
   [7-2] The insulin-producing cells (cell population) according to [7-1], wherein the pancreatic islet-like cells comprise chromogranin A-positive cells at a proportion of 50% or more.
   [7-2-1] The insulin-producing cells (cell population) according to [7-1], wherein the pancreatic islet-like cells comprise chromogranin A-positive cells at a proportion of 95% or more.
   [7-3] The insulin-producing cells (cell population) according to any of [7-1] to [7-2-1], wherein the pancreatic islet-like cells comprise Ki67-positive cells at a proportion of less than 3%.
   [7-3-1] The insulin-producing cells (cell population) according to any of [7-1] to [7-2-1], wherein the pancreatic islet-like cells comprise Ki67-positive cells at a proportion of less than 1%.
   [7-4] The insulin-producing cells (cell population) according to any of [7-1] to [7-3-1], wherein the pancreatic islet-like cells comprise glucagon-positive and insulin-negative cells at a proportion of 10% or more.
   [7-5] The insulin-producing cells (cell population) according to any of [7-1] to [7-4], wherein the pancreatic islet-like cells exhibit insulin-secreting action in response to hypoglycemia.
[8] A medicament comprising the insulin-producing cells according to any of [1] to [7-5].
[9] The medicament according to [8], wherein the insulin-producing cells (cell population) are accommodated in a device.
[10] The medicament according to [8] or [9], wherein the insulin-producing cells (cell population) are dispersed in a hydrogel.
[11] The medicament according to any of [8] to [10], to be used for transplantation into a living body.
[12] The medicament according to any of [8] to [11], to be used for subcutaneous transplantation.
[13] The medicament according to any of [8] to [12], for use in treatment of diabetes mellitus.
[14] The medicament according to any of [8] to [12], for use in improvement and/or retention of control of fasting and postprandial glucose levels in a patient.
[15] The medicament according to any of [8] to [12], to be used to reduce risk of hypoglycemia in a patient with diabetes mellitus.
[16] The medicament according to any of [8] to [12], for use in a method for inducing differentiation into pancreatic islet-like cells (cell population) in a living body.
[17] The medicament according to [16], wherein pancreatic islet-like cells (cell population) differentiated in a living body comprise chromogranin A-positive cells at a proportion of 50% or more.
   [17-1] The medicament according to [16], wherein pancreatic islet-like cells (cell population) differentiated in a living body comprise chromogranin A-positive cells at a proportion of 95% or more.
[18] The medicament according to any of [16] to [17-1], wherein pancreatic islet-like cells (cell population) differentiated in a living body comprise Ki67-positive cells at a proportion of less than 3%.
[18] The medicament according to any of [16] to [17-1], wherein pancreatic islet-like cells (cell population) differentiated in a living body comprise Ki67-positive cells at a proportion of less than 1%.
[19] The medicament according to any of [16] to [18], wherein pancreatic islet-like cells (cell population) differentiated in a living body comprise glucagon-positive and insulin-negative cells at a proportion of 10% or more.
[20] The medicament according to any of [16] to [19], wherein pancreatic islet-like cells (cell population) differentiated in a living body exhibit insulin-secreting action in response to hypoglycemia.
[21] A method for producing insulin-producing cells (cell population) comprising: insulin-positive and NKX6.1-positive cells at a proportion of 30% or more; and insulin-positive and NKX6.1-negative cells at a proportion of more than 15%, the method comprising the steps of:
   culturing pluripotent stem cells in the presence of a low dose of activin A to produce definitive endoderm cells (cell population); and
   culturing endocrine progenitor cells in a medium containing an FGFR1 inhibitor to produce the insulin-producing cells (cell population).
[22] A method for generating pancreatic islet-like cells (cell population), the method comprising
   transplanting insulin-producing cells (cell population) into a living body to induce differentiation into pancreatic islet-like cells (cell population), wherein the insulin-producing cells comprise: insulin-positive and NKX6.1-positive cells in a proportion of 30% or more; and insulin-positive and NKX6.1-negative cells in a proportion of more than 15%.
[23] A method for treating diabetes mellitus, the method comprising
   transplanting insulin-producing cells (cell population) into a living body to induce differentiation into pancreatic islet-like cells (cell population), wherein the insulin-producing cells comprise: insulin-positive and NKX6.1-positive cells in a proportion of 30% or more; and insulin-positive and NKX6.1-negative cells in a proportion of more than 15%.
[24] A method for improving and/or retaining control of fasting and postprandial glucose levels in a patient, the method comprising
   transplanting insulin-producing cells (cell population) into a living body to induce differentiation into pancreatic islet-like cells (cell population), wherein the insulin-producing cells comprise: insulin-positive and NKX6.1-positive cells in a proportion of 30% or more; and insulin-positive and NKX6.1-negative cells in a proportion of more than 15%.
[25] A method for reducing risk of hypoglycemia in a patient with diabetes mellitus, the method comprising:
   transplanting insulin-producing cells (cell population) into a living body to induce differentiation into pancreatic islet-like cells (cell population), wherein the insulin-producing cells comprise: insulin-positive and NKX6.1-positive cells in a proportion of 30% or more; and insulin-positive and NKX6.1-negative cells in a proportion of more than 15%.
[26] The insulin-producing cells (cell population) according to any of [1] to [7-5], for use in a method for treating diabetes mellitus.
[27] The insulin-producing cells (cell population) according to any of [1] to [7-5], for use in a method for improving and/or retaining control of fasting and postprandial glucose levels in a patient.
[28] The insulin-producing cells (cell population) according to any of [1] to [7-5], for use in a method for reducing risk of hypoglycemia in a patient with diabetes mellitus.
[29] The insulin-producing cells (cell population) according to any of [1] to [7-5], for use in a method for inducing differentiation into pancreatic islet-like cells (cell population) in a living body.
[30] The insulin-producing cells (cell population) according to any of [26] to [29], wherein the insulin-producing cells (cell population) are accommodated in a device.
[31] The insulin-producing cells (cell population) according to any of [26] to [30], wherein the insulin-producing cells (cell population) are dispersed in a hydrogel.
[32] The insulin-producing cells (cell population) according to any of [26] to [31], to be used for transplantation into a living body.
[33] The insulin-producing cells (cell population) according to any of [26] to [32], to be used for subcutaneous transplantation.
[34] The insulin-producing cells (cell population) according to [29], wherein pancreatic islet-like cells (cell population) differentiated in a living body comprise chromogranin A-positive cells at a proportion of 50% or more.
   [34-1] The insulin-producing cells (cell population) according to [29], wherein pancreatic islet-like cells (cell population) differentiated in a living body comprise chromogranin A-positive cells at a proportion of 95% or more.
[35] The insulin-producing cells (cell population) according to [29], wherein pancreatic islet-like cells (cell population) differentiated in a living body comprise Ki67-positive cells at a proportion of less than 3%.
   [35-1] The insulin-producing cells (cell population) according to [29], wherein pancreatic islet-like cells (cell population) differentiated in a living body comprise Ki67-positive cells at a proportion of less than 1%.
   [35-2] The insulin-producing cells (cell population) according to [29], wherein pancreatic islet-like cells (cell population) differentiated in a living body comprise glucagon-positive and insulin-negative cells at a proportion of 10% or more.
[36] The insulin-producing cells (cell population) according to [29], wherein pancreatic islet-like cells (cell population) differentiated in a living body exhibit insulin-secreting action in response to hypoglycemia.
[37] Use of the insulin-producing cells (cell population) according to any of [1] to [7-5] in the manufacture of a medicament for treating diabetes mellitus.
[38] Use of the insulin-producing cells (cell population) according to any of [1] to [7-5] in the manufacture of a medicament for improving and/or retaining control of fasting and postprandial glucose levels in a patient.
[39] Use of the insulin-producing cells (cell population) according to any of [1] to [7-5] in the manufacture of a medicament for reducing risk of hypoglycemia in a patient with diabetes mellitus.

The present specification encompasses the contents described in the specification and/or drawings of Japanese Patent Application No. 2018-175465 on which the priority of the present application is based.

All the publications, patents, and patent applications cited herein are totally incorporated herein by reference.

### Advantageous Effects of Invention

The present invention can provide a novel approach that enables efficient induction/production of endocrine cells from pluripotent stem cells.

### Brief Description of Drawings

[Figure 1] Figure 1 shows results of flow cytometry measurement of expressions of insulin (INS), NKX6.1, glucagon (GCG), and Ki67 in the prototype of insulin-producing cells (prototype), insulin-producing cells, and the human pancreatic islet (islet).
[Figure 2] Figure 2 shows results of measurement of gene expression levels for insulin (INS), glucagon (GCG), MafA, and UCN3 in the prototype of insulin-producing cells (prototype), insulin-producing cells, and the human pancreatic islet (islet) by a quantitative PCR method.
[Figure 3] Figure 3 shows a graph representing the number of iPS cell colonies retaining an undifferentiated state in insulin-producing cells prepared by spiking with 30 (0.005%) iPS cells, 6 (0.001%) iPS cells, and 0 iPS cells retaining an undifferentiated state.
[Figure 4] Figure 4 shows a phase-contrast microscopic image of a cell aggregate obtained by thawing cryopreserved insulin-producing cells and then culturing the insulin-producing cells by three-dimensional culture for 4 days.
[Figure 5] Figure 5 shows results of flow cytometry measurement of expressions of INS, NKX6.1, and Ki67 in insulin-producing cells before cryopreservation and insulin-producing cells after cryopreservation and thawing.
[Figure 6] Figure 6 shows results of measurement of human C-peptide concentrations in blood after glucose loading in diabetes mellitus mice with transplanted insulin-producing cells and non-diabetes mellitus mice without transplanted insulin-producing cells.
[Figure 7] Figure 7 shows results of measurement of human C-peptide concentrations in blood and glucagon concentrations in blood after administration of glargine in diabetes mellitus mice with transplanted insulin-producing cells and non-diabetes mellitus mice without transplanted insulin-producing cells.
[Figure 8] Figure 8 shows results of flow cytometry measurement of expressions of insulin, NKX6.1, glucagon, and chromogranin A in subcutaneously transplanted insulin-producing cells.
[Figure 9] Figure 9 shows results of immunohistological staining for insulin and glucagon in subcutaneously transplanted insulin-producing cells. Green: insulin-positive cells. Red: glucagon-positive cells.

### Description of Embodiments

### 1. Terminology

Hereinafter, the terms described herein will be explained.

As used herein, "about" refers to a value which may vary up to plus or minus 25%, 20%, 10%, 8%, 6%, 5%, 4%, 3%, 2%, or 1% from the reference value. Preferably, the term "about" or "around" refers to a range from minus or plus 15%, 10%, 5%, or 1% from the reference value.

Each numerical range specifying the present invention herein includes numerical values substantially regarded as the lower limit value and upper limit value of the numerical range, even when "about" is not stated.

As used herein, "comprise(s)" or "comprising" means inclusion of the element(s) following the word without limitation thereto. Accordingly, it indicates inclusion of the element(s) following the word, but does not indicate exclusion of any other element.

As used herein, "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist.

As used herein, "without the use of feeder cell(s)" means basically containing no feeder cells and using no medium preconditioned by culturing feeder cells. Accordingly, the medium does not contain any substance, such as a growth factor or a cytokine, secreted by feeder cells.

"Feeder cells" or "feeder" means cells that are co-cultured with another kind of cells, support the cells, and provide an environment that allows the cells to grow. The feeder cells may be derived from the same species as or a different species from the cells that they support. For example, as a feeder for human cells, human skin fibroblasts or human embryonic-stem cells may be used or a primary culture of murine embryonic fibroblasts or immortalized murine embryonic fibroblasts may be used. The feeder cells can be inactivated by exposure to radiation or treatment with mitomycin C.

As used herein, "adhered (adherent, adhering, adhesion)" refers to cells are attached to a container, for example, cells are attached to a cell culture dish or a flask made of a sterilized plastic (or coated plastic) in the presence of an appropriate medium. Some cells cannot be maintained or grow in culture without adhering to the cell culture container. In contrast, non-adherent cells can be maintained and proliferate in culture without adhering to the container.

As used herein, "culture" refers to maintaining, growing, and/or differentiating cells in in vitro environment. "Culturing" means maintaining, proliferating (growing), and/or differentiating cells out of tissue or the living body, for example, in a cell culture dish or flask. The culture includes two-dimensional culture (plane culture) and three-dimensional culture (suspension culture).

As used herein, "enrich(es)" and "enrichment" refer to increasing the amount of a certain component in a composition such as a composition of cells and "enriched" refers, when used to describe a composition of cells, for example, a cell population, to a cell population increased in the amount of a certain component in comparison with the percentage of such component in the cell population before the enrichment. For example, a composition such as a cell population can be enriched for a target cell type and, accordingly, the percentage of the target cell type is increased in comparison with the percentage of the target cells present in the cell population before the enrichment. A cell population can be enriched for a target cell type by a method of selecting and sorting cells known in the art. A cell population can be enriched by a specific process of sorting or selection described herein. In a certain embodiment of the present invention, a cell population is enriched for a target cell population at least 20%, 30%, 40%, 50%, 60%, 70%, 80%, 85%, 90%, 95%, 97%, 98%, or 99% by a method of enriching the target cell population.

As used herein, "deplete(s)" and "depletion" refer to decreasing the amount of a certain component in cells or a composition such as a composition of cells and "depleted" refers, when used to describe cells or a composition of cells, for example, a cell population, to a cell population decreased in the amount of a certain component in comparison with the percentage of such component in the cell population before the depletion. For example, a composition such as a cell population can be depleted for a target cell type and, accordingly, the percentage of the target cell type is decreased in comparison with the percentage of the target cells present in the cell population before the depletion. A cell population can be depleted for a target cell type by a method of selecting and sorting cells known in the art. A cell population can be depleted by a specific process of sorting or selection described herein. In a certain embodiment of the present invention, a cell population is reduced (depleted) for a target cell population at least 50%, 80%, 85%, 90%, 95%, 97%, 98%, or 99% by a method of depleting a target cell population.

As used herein, "purify(ies)" and "purification" refer to removing impurities in a composition such as a composition of cells and making it pure for a certain component and "purified" refers, when used to describe a composition of cells, for example, a cell population, to a cell population in which the amount of impurities is decreased in comparison with the percentage of such components in the cell population before purification and the purity of a certain component is improved. For example, a composition such as a cell population can be purified for a target cell type and, accordingly, the percentage of the target cell type is increased in comparison with the percentage of the target cells present in the cell population before the purification. A cell population can be purified for a target cell type by a method of selecting and sorting cells known in the art. A cell population can be purified by a specific process of sorting or selection described herein. In a certain embodiment of the present invention, the purity of a target cell population is brought by a method of purifying a target cell population to at least 70%, 80%, 85%, 90%, 95%, 97%, 98%, or 99% or to the extent at which impurities (including contaminant cells) are undetectable.

As used herein, "factor having CDK8/19-inhibiting activity" means any substance having the inhibitory activity for CDK8/19. CDK8, in contrast to the other proteins of the same CDK family, is not required for cell proliferation. The inhibition of CDK8 has no great effect under usual conditions. CDK19 and CDK8 are similar to each other. Usually, the inhibition of CDK8 also involves the inhibition of CDK19.

"Growth factor" is an endogenous protein that promotes differentiation and/or proliferation of particular cells. Examples of "growth factor" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transformation growth factor beta (TGF-β), vascular endothelial growth factor (VEGF), transferrin, various interleukins (for example, IL-1 to IL-18), various colony-stimulating factors (for example, granulocyte/macrophage colony-stimulating factor (GM-CSF)), various interferons (for example, IFN-γ, and the like), and other cytokines having effects on stem cells, for example, stem cell factor (SCF), and erythropoietin (Epo).

As used herein, "ROCK inhibitor" means a substance that inhibits Rho kinase (ROCK: Rho-associated, coiled-coil containing protein kinase) and may be a substance that inhibits either of ROCK I and ROCK II. The ROCK inhibitor is not particularly limited as long as it has the aforementioned function and examples include N-(4-pyridinyl)-4β-[(R)-1-aminoethyl]cyclohexane-1α-carboxamide (that may be also referred to as Y-27632), Fasudil (HA1077), (2S)-2-methyl-l-[(4-methyl-5-isoquinolinyl)sulfonyl]hexahydro-1H-1,4-diazepine (H-1152), 4β-[(1R)-1-aminoethyl]-N-(4-pyridyl)benzene-1zencarboxamide (Wf-536), N-(1H-pyrrolo[2,3-b]pyridin-4-yl)-4β-[(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), N-(6-fluoro-1H-indazol-5-yl)-6-methyl-2-oxo-4-[4-(trifluoromethyl)phenyl]-3,4-dihydro-1H-pyridine-5-carboxamide (GSK429286A). The ROCK inhibitor is not limited to these and antisense oligonucleotides and siRNA to ROCK mRNA, antibodies that bind to ROCK, and dominant negative ROCK mutants can also be used as a ROCK inhibitor, and commercially available, or synthesized according to a known method.

As used herein, "GSK3β inhibitor" is a substance having the inhibitory activity for GSK3β (glycogen synthase kinase 3β). GSK3 (glycogen synthase kinase 3) is a serine/threonine protein kinase and involved in many signaling pathways associated with the production of glycogen, apoptosis, maintenance of stem cells, etc. GSK3 has the 2 isoforms α and β. "GSK3β inhibitor" used in the present invention is not particularly limited as long as it has the GSK3β-inhibiting activity and it may be a substance having both the GSK3α-inhibiting activity and the GSK3β-inhibiting activity.

Examples of GSK3β inhibitor include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), and AR-AO144-18, CT99021, CT20026, BIO, BIO-acetoxime, pyridocarbazole-ruthenium cyclopentadienyl complex, OTDZT, alpha-4-dibromoacetophenone, lithium, and the like. GSK3β is not limited to these and antisense oligonucleotides and siRNA to GSK3β mRNA, antibodies that bind to GSK3β, dominant negative GSK3β mutants, and the like can also be used as GSK3β inhibitor, and commercially available, or synthesized according to a known method.

As used herein, examples of "serum replacement" include Knockout Serum Replacement (KSR: Invitrogen), StemSure Serum Replacement (Wako), B-27 supplement, N2-supplement, albumin (for example, lipid rich albumin), insulin, transferrin, fatty acids, collagen precursors, trace elements (for example, zinc, selenium (for example, sodium selenite)), 2-mercaptoethanol, 3'-thiolglycerol, or mixtures thereof (for example, ITS-G). Preferred serum replacements are B-27 supplement, KSR, StemSure Serum Replacement, ITS-G. The concentration of serum replacement in a medium when added into a medium is 0.01-10% by weight, and preferably 0.1-2% by weight. In the present invention, "serum replacement" is preferably used instead of serum.

As used herein, "FGFR1 inhibitor" is a substance having inhibitory activity for fibroblast growth factor receptor (FGFR) 1. FGFR1 is a member of the four-pass transmembrane tyrosine kinase family (FGFR1, FGFR2, FGFR3, and FGFR4), as a receptor having high affinity for growth factors FGF1 to FGF17. The FGFR1 inhibitor is not particularly limited as long as the FGFR1 inhibitor has FGFR1-inhibiting activity. The FGFR1 inhibitor may be a substance having the FGFR1-inhibiting activity as well as inhibitory activity for other FGFRs. In the present specification, "FGFR1 inhibitor" includes a substance having FGFR1-inhibiting activity, even if only slightly, and preferably refers to a substance that inhibits FGFR1 by 50% or more, more preferably a substance having a 50% inhibitory concentration (IC₅₀) of 1 µM or lower, further preferably 100 nM or lower, against FGFR1. A method for determining the FGFR1-inhibiting activity can be selected from known methods. Examples thereof include determination methods using EnzyChrom Kinase Assay Kit (BioAssay Systems). A conventionally known FGFR1 inhibitor may be used and can be found in patent literatures or non patent literatures.

As used herein, examples of the "FGFR1 inhibitor" include compounds given below (compound group D) as well as compounds having inhibitory activity for FGFR1 (or salts thereof) among compounds having structural formulas represented by the general formulas given below. The FGFR1 inhibitor is preferably a compound having a 50% inhibitory concentration (IC₅₀) of 1 µM or lower, more preferably 100 nM or lower, against FGFR1 (or a salt thereof) among compound I and compound II having structural formulas represented by the following general formulas.

### (Compound I)

Examples of compound I include compounds represented by the following formula 1: 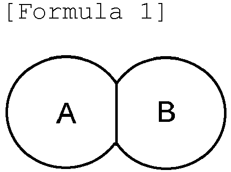 wherein ring AB shows a bicyclic heterocycle having one or more substituent(s) (ring AB may be a tricyclic heterocycle having one or more substituent(s)) or salts thereof.

Preferably, in the formula, ring AB shows a bicyclic nitrogen-containing heterocycle having three substituents.

Examples of the substituents include substituents given below and substituents of compound group D. Substituents of compound group D are preferred.

### (Compound II)

Examples of compound II include compounds represented by the following formula 2: 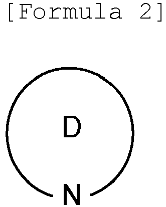
wherein ring D shows a 5- or 6-membered monocyclic nitrogen-containing heterocycle optionally having substituent(s); and
one or more nitrogen-containing heterocycle(s) other than the ring D is contained
or salts thereof.

Preferably, in the formula, ring D shows an aromatic ring containing one or two nitrogen atoms and optionally having substituent(s).

Preferably, examples of the one or more nitrogen-containing heterocycle(s) other than the ring D include piperazine optionally having substituent(s).

Examples of the substituents include substituents given below and substituents of compound group D. Substituents of compound group D are preferred.

As used herein, examples of the "heterocycle" include aromatic heterocycles and non-aromatic heterocycles each containing, as a ring-constituting atom besides carbon atom, 1 to 4 hetero atoms selected from a nitrogen atom, a sulfur atom and an oxygen atom.

As used herein, examples of the "aromatic heterocycle" include a 5- to 14-membered (preferably 5-to 10-membered) aromatic heterocycle containing, as a ring-constituting atom besides carbon atom, 1 to 4 hetero atoms selected from a nitrogen atom, a sulfur atom and an oxygen atom. Preferable examples of the "aromatic heterocycle" include 5- or 6-membered monocyclic aromatic heterocycles such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, and triazine; and 8- to 14-membered fused polycyclic (preferably bicyclic or tricyclic) aromatic heterocycles such as benzothiophene, benzofuran, benzimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiin, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, and phenoxazine.

As used herein, examples of the "non-aromatic heterocycle" include a 3- to 14-membered (preferably 4-to 10-membered) non-aromatic heterocycle containing, as a ring-constituting atom besides carbon atom, 1 to 4 hetero atoms selected from a nitrogen atom, a sulfur atom and an oxygen atom. Preferable examples of the "non-aromatic heterocycle" include 3- to 8-membered monocyclic non-aromatic heterocycles such as aziridine, oxirane, thiirane, azetidine, oxetane, thietane, tetrahydrothiophene, tetrahydrofuran, pyrroline, pyrrolidine, imidazoline, imidazolidine, oxazoline, oxazolidine, pyrazoline, pyrazolidine, thiazoline, thiazolidine, tetrahydroisothiazole, tetrahydrooxazole, tetrahydroisoxazole, piperidine, piperazine, tetrahydropyridine, dihydropyridine, dihydrothiopyran, tetrahydropyrimidine, tetrahydropyridazine, dihydropyran, tetrahydropyran, tetrahydrothiopyran, morpholine, thiomorpholine, azepanine, diazepane, azepine, azocane, diazocane, oxepane; and
9- to 14-membered fused polycyclic (preferably bi or tricyclic) non-aromatic heterocycles such as dihydrobenzofuran, dihydrobenzimidazole, dihydrobenzoxazole, dihydrobenzothiazole, dihydrobenzisothiazole, dihydronaphtho[2,3-b]thiophene, tetrahydroisoquinoline, tetrahydroquinoline, 4H-quinolizine, indoline, isoindoline, tetrahydrothieno[2,3-c]pyridine, tetrahydrobenzazepine, tetrahydroquinoxaline, tetrahydrophenanthridine, hexahydrophenothiazine, hexahydrophenoxazine, tetrahydrophthalazine, tetrahydronaphthyridine, tetrahydroquinazoline, tetrahydrocinnoline, tetrahydrocarbazole, tetrahydro-β-carboline, tetrahydroacridine, tetrahydrophenazine, tetrahydrothioxanthene, octahydroisoquinoline.

As used herein, examples of the "nitrogen-containing heterocycle" include "heterocycles" containing at least one or more nitrogen atom(s) as a ring-constituting atom.

Hereinafter, each substituent used herein will be defined in detail. Each substituent is as defined below unless otherwise specified.

As used herein, examples of the "halogen atom" include fluorine, chloride, bromine, and iodine.

As used herein, examples of the "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

As used herein, examples of the "optionally halogenated C₁₋₆ alkyl group" include a C₁₋₆ alkyl group optionally having 1 to 7, preferably 1 to 5 halogen atoms. Specific examples thereof include methyl, chloromethyl, difluoromethyl, trichloromethyl, trifluoromethyl, ethyl, 2-bromoethyl, 2,2,2-trifluoroethyl, tetrafluoroethyl, pentafluoroethyl, propyl, 2,2-difluoropropyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, and 6,6,6-trifluorohexyl.

As used herein, examples of the "C₂₋₆ alkenyl group" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, and 5-hexenyl.

As used herein, examples of the "C₂₋₆ alkynyl group" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, and 4-methyl-2-pentynyl.

As used herein, examples of the "C₃₋₁₀ cycloalkyl group" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, and adamantyl.

As used herein, examples of the "optionally halogenated C₃₋₁₀ cycloalkyl group" include a C₃₋₁₀ cycloalkyl group optionally having 1 to 7, preferably 1 to 5 halogen atoms. Specific examples thereof include cyclopropyl, 2,2-difluorocyclopropyl, 2,3-difluorocyclopropyl, cyclobutyl, difluorocyclobutyl, cyclopentyl, cyclohexyl, cycloheptyl, and cyclooctyl.

As used herein, examples of the "C₃₋₁₀ cycloalkenyl group" include cyclopropenyl, cyclobutenyl, cyclopentenyl, cyclohexenyl, cycloheptenyl, and cyclooctenyl.

As used herein, examples of the "C₆₋₁₄ aryl group" include phenyl, 1-naphthyl, 2-naphthyl, 1-anthryl, 2-anthryl, and 9-anthryl.

As used herein, examples of the "C₇₋₁₆ aralkyl group" include benzyl, phenethyl, naphthylmethyl, and phenylpropyl.

As used herein, examples of the "C₁₋₆ alkoxy group" include methoxy, ethoxy, propoxy, isopropoxy, butoxy, isobutoxy, sec-butoxy, tert-butoxy, pentyloxy, and hexyloxy.

As used herein, examples of the "optionally halogenated C₁₋₆ alkoxy group" include a C₁₋₆ alkoxy group optionally having 1 to 7, preferably 1 to 5 halogen atoms. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, and hexyloxy.

As used herein, examples of the "C₃₋₁₀ cycloalkyloxy group" include cyclopropyloxy, cyclobutyloxy, cyclopentyloxy, cyclohexyloxy, cycloheptyloxy, and cyclooctyloxy.

As used herein, examples of the "C₁₋₆ alkylthio group" include methylthio, ethylthio, propylthio, isopropylthio, butylthio, sec-butylthio, tert-butylthio, pentylthio, and hexylthio.

As used herein, examples of the "optionally halogenated C₁₋₆ alkylthio group" include a C₁₋₆ alkylthio group optionally having 1 to 7, preferably 1 to 5 halogen atoms. Specific examples thereof include methylthio, difluoromethylthio, trifluoromethylthio, ethylthio, propylthio, isopropylthio, butylthio, 4,4,4-trifluorobutylthio, pentylthio, and hexylthio.

As used herein, examples of the "C₁₋₆ alkyl-carbonyl group" include acetyl, propanoyl, butanoyl, 2-methylpropanoyl, pentanoyl, 3-methylbutanoyl, 2-methylbutanoyl, 2,2-dimethylpropanoyl, hexanoyl, and heptanoyl.

As used herein, examples of the "optionally halogenated C₁₋₆ alkyl-carbonyl group" include a C₁₋₆ alkyl-carbonyl group optionally having 1 to 7, preferably 1 to 5 halogen atoms. Specific examples thereof include acetyl, chloroacetyl, trifluoroacetyl, trichloroacetyl, propanoyl, butanoyl, pentanoyl, and hexanoyl.

As used herein, examples of the "C₁₋₆ alkoxy-carbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, and hexyloxycarbonyl.

As used herein, examples of the "C₆₋₁₄ aryl-carbonyl group" include benzoyl, 1-naphthoyl, and 2-naphthoyl.

As used herein, examples of the "C₇₋₁₆ aralkyl-carbonyl group" include phenylacetyl and phenylpropionyl.

As used herein, examples of the "5- to 14-membered aromatic heterocyclylcarbonyl group" include nicotinoyl, isonicotinoyl, thenoyl, and furoyl.

As used herein, examples of the "3- to 14-membered non-aromatic heterocyclylcarbonyl group" include morpholinylcarbonyl, piperidinylcarbonyl, and pyrrolidinylcarbonyl.

As used herein, examples of the "mono- or di-C₁₋₆ alkyl-carbamoyl group" include methylcarbamoyl, ethylcarbamoyl, dimethylcarbamoyl, diethylcarbamoyl, and N-ethyl-N-methylcarbamoyl.

As used herein, examples of the "mono- or di-C₇₋₁₆ aralkyl-carbamoyl group" include benzylcarbamoyl and phenethylcarbamoyl.

As used herein, examples of the "C₁₋₆ alkylsulfonyl group" include methylsulfonyl, ethylsulfonyl, propylsulfonyl, isopropylsulfonyl, butylsulfonyl, sec-butylsulfonyl, and tert-butylsulfonyl.

As used herein, examples of the "optionally halogenated C₁₋₆ alkylsulfonyl group" include a C₁₋₆ alkylsulfonyl group optionally having 1 to 7, preferably 1 to 5 halogen atoms. Specific examples thereof include methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl, and hexylsulfonyl.

As used herein, examples of the "C₆₋₁₄ arylsulfonyl group" include phenylsulfonyl, 1-naphthylsulfonyl, and 2-naphthylsulfonyl.

As used herein, examples of the "substituent" include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

In the present specification, examples of the "hydrocarbon group" (including "hydrocarbon group" of "optionally substituted hydrocarbon group") include a C₁₋₆ alkyl group, a C₂₋₆ alkenyl group, a C₂₋₆ alkynyl group, a C₃₋₁₀ cycloalkyl group, a C₃₋₁₀ cycloalkenyl group, a C₆₋₁₄ aryl group and a C₇₋₁₆ aralkyl group.

In the present specification, examples of the "optionally substituted hydrocarbon group" include a hydrocarbon group optionally having substituent(s) selected from the following substituent group A.

### [substituent group A]

(1) a halogen atom,
(2) a nitro group,
(3) a cyano group,
(4) an oxo group,
(5) a hydroxy group,
(6) an optionally halogenated C₁₋₆ alkoxy group,
(7) a C₆₋₁₄ aryloxy group (e.g., phenoxy, naphthoxy),
(8) a C₇₋₁₆ aralkyloxy group (e.g., benzyloxy),
(9) a 5- to 14-membered aromatic heterocyclyloxy group (e.g., pyridyloxy),
(10) a 3- to 14-membered non-aromatic heterocyclyloxy group (e.g., morpholinyloxy, piperidinyloxy),
(11) a C₁₋₆ alkyl-carbonyloxy group (e.g., acetoxy, propanoyloxy),
(12) a C₆₋₁₄ aryl-carbonyloxy group (e.g., benzoyloxy, 1-naphthoyloxy, 2-naphthoyloxy),
(13) a C₁₋₆ alkoxy-carbonyloxy group (e.g., methoxycarbonyloxy, ethoxycarbonyloxy, propoxycarbonyloxy, butoxycarbonyloxy),
(14) a mono- or di-C₁₋₆ alkyl-carbamoyloxy group (e.g., methylcarbamoyloxy, ethylcarbamoyloxy, dimethylcarbamoyloxy, diethylcarbamoyloxy),
(15) a C₆₋₁₄ aryl-carbamoyloxy group (e.g., phenylcarbamoyloxy, naphthylcarbamoyloxy),
(16) a 5- to 14-membered aromatic heterocyclylcarbonyloxy group (e.g., nicotinoyloxy),
(17) a 3- to 14-membered non-aromatic heterocyclylcarbonyloxy group (e.g., morpholinylcarbonyloxy, piperidinylcarbonyloxy),
(18) an optionally halogenated C₁₋₆ alkylsulfonyloxy group (e.g., methylsulfonyloxy, trifluoromethylsulfonyloxy),
(19) a C₆₋₁₄ arylsulfonyloxy group optionally substituted by a C₁₋₆ alkyl group (e.g., phenylsulfonyloxy, toluenesulfonyloxy),
(20) an optionally halogenated C₁₋₆ alkylthio group,
(21) a 5- to 14-membered aromatic heterocyclic group,
(22) a 3- to 14-membered non-aromatic heterocyclic group,
(23) a formyl group,
(24) a carboxy group,
(25) an optionally halogenated C₁₋₆ alkyl-carbonyl group,
(26) a C₆₋₁₄ aryl-carbonyl group,
(27) a 5- to 14-membered aromatic heterocyclylcarbonyl group,
(28) a 3- to 14-membered non-aromatic heterocyclylcarbonyl group,
(29) a C₁₋₆ alkoxy-carbonyl group,
(30) a C₆₋₁₄ aryloxy-carbonyl group (e.g., phenyloxycarbonyl, 1-naphthyloxycarbonyl, 2-naphthyloxycarbonyl),
(31) a C₇₋₁₆ aralkyloxy-carbonyl group (e.g., benzyloxycarbonyl, phenethyloxycarbonyl),
(32) a carbamoyl group,
(33) a thiocarbamoyl group,
(34) a mono- or di-C₁₋₆ alkyl-carbamoyl group,
(35) a C₆₋₁₄ aryl-carbamoyl group (e.g., phenylcarbamoyl),
(36) a 5- to 14-membered aromatic heterocyclylcarbamoyl group (e.g., pyridylcarbamoyl, thienylcarbamoyl),
(37) a 3- to 14-membered non-aromatic heterocyclylcarbamoyl group (e.g., morpholinylcarbamoyl, piperidinylcarbamoyl),
(38) an optionally halogenated C₁₋₆ alkylsulfonyl group,
(39) a C₆₋₁₄ arylsulfonyl group,
(40) a 5- to 14-membered aromatic heterocyclylsulfonyl group (e.g., pyridylsulfonyl, thienylsulfonyl),
(41) an optionally halogenated C₁₋₆ alkylsulfinyl group,
(42) a C₆₋₁₄ arylsulfinyl group (e.g., phenylsulfinyl, 1-naphthylsulfinyl, 2-naphthylsulfinyl),
(43) a 5- to 14-membered aromatic heterocyclylsulfinyl group (e.g., pyridylsulfinyl, thienylsulfinyl),
(44) an amino group,
(45) a mono- or di-C₁₋₆ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di-C₆₋₁₄ arylamino group (e.g., phenylamino),
(47) a 5- to 14-membered aromatic heterocyclylamino group (e.g., pyridylamino),
(48) a C₇₋₁₆ aralkylamino group (e.g., benzylamino),
(49) a formylamino group,
(50) a C₁₋₆ alkyl-carbonylamino group (e.g., acetylamino, propanoylamino, butanoylamino),
(51) a (C₁₋₆ alkyl) (C₁₋₆ alkyl-carbonyl) amino group (e.g., N-acetyl-N-methylamino),
(52) a C₆₋₁₄ aryl-carbonylamino group (e.g., phenylcarbonylamino, naphthylcarbonylamino),
(53) a C₁₋₆ alkoxy-carbonylamino group (e.g., methoxycarbonylamino, ethoxycarbonylamino, propoxycarbonylamino, butoxycarbonylamino, tert-butoxycarbonylamino),
(54) a C₇₋₁₆ aralkyloxy-carbonylamino group (e.g., benzyloxycarbonylamino),
(55) a C₁₋₆ alkylsulfonylamino group (e.g., methylsulfonylamino, ethylsulfonylamino),
(56) a C₆₋₁₄ arylsulfonylamino group optionally substituted by a C₁₋₆ alkyl group (e.g., phenylsulfonylamino, toluenesulfonylamino),
(57) an optionally halogenated C₁₋₆ alkyl group,
(58) a C₂₋₆ alkenyl group,
(59) a C₂₋₆ alkynyl group,
(60) a C₃₋₁₀ cycloalkyl group,
(61) a C₃₋₁₀ cycloalkenyl group and
(62) a C₆₋₁₄ aryl group.

The number of the above-mentioned substituents in the "optionally substituted hydrocarbon group" is, for example, 1 to 5, preferably 1 to 3. When the number of the substituents is two or more, the respective substituents may be the same or different.

In the present specification, examples of the "heterocyclic group" (including "heterocyclic group" of "optionally substituted heterocyclic group") include (i) an aromatic heterocyclic group, (ii) a non-aromatic heterocyclic group and (iii) a 7- to 10-membered bridged heterocyclic group, each containing, as a ring-constituting atom besides carbon atom, 1 to 4 hetero atoms selected from a nitrogen atom, a sulfur atom and an oxygen atom.

In the present specification, examples of the "aromatic heterocyclic group" (including "5- to 14-membered aromatic heterocyclic group") include a 5- to 14-membered (preferably 5- to 10-membered) aromatic heterocyclic group containing, as a ring-constituting atom besides carbon atom, 1 to 4 hetero atoms selected from a nitrogen atom, a sulfur atom and an oxygen atom.

Preferable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and
8- to 14-membered fused polycyclic (preferably bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

In the present specification, examples of the "non-aromatic heterocyclic group" (including "3- to 14-membered non-aromatic heterocyclic group") include a 3-to 14-membered (preferably 4- to 10-membered) non-aromatic heterocyclic group containing, as a ring-constituting atom besides carbon atom, 1 to 4 hetero atoms selected from a nitrogen atom, a sulfur atom and an oxygen atom.

Preferable examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and
9- to 14-membered fused polycyclic (preferably bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, preferable examples of the "7- to 10-membered bridged heterocyclic group" include quinuclidinyl and 7-azabicyclo[2.2.1]heptanyl.

In the present specification, examples of the "nitrogen-containing heterocyclic group" include a "heterocyclic group" containing at least one nitrogen atom as a ring-constituting atom.

In the present specification, examples of the "optionally substituted heterocyclic group" include a heterocyclic group optionally having substituent(s) selected from the aforementioned substituent group A.

The number of the substituents in the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each optionally having "1 or 2 substituents selected from a C₁₋₆ alkyl group, a C₂₋₆ alkenyl group, a C₃₋₁₀ cycloalkyl group, a C₃₋₁₀ cycloalkenyl group, a C₆₋₁₄ aryl group, a C₇₋₁₆ aralkyl group, a 5- to 14-membered aromatic heterocyclic group and a 3- to 14-membered non-aromatic heterocyclic group, each of which optionally has 1 to 3 substituents selected from a halogen atom, an optionally halogenated C₁₋₆ alkoxy group, a hydroxy group, a nitro group, a cyano group, an amino group and a carbamoyl group".

Examples of the "acyl group" also include a hydrocarbon-sulfonyl group, a heterocyclylsulfonyl group, a hydrocarbon-sulfinyl group and a heterocyclylsulfinyl group.

Here, the hydrocarbon-sulfonyl group means a hydrocarbon group-bonded sulfonyl group, the heterocyclylsulfonyl group means a heterocyclic group-bonded sulfonyl group, the hydrocarbon-sulfinyl group means a hydrocarbon group-bonded sulfinyl group and the heterocyclylsulfinyl group means a heterocyclic group-bonded sulfinyl group.

Preferable examples of the "acyl group" include a formyl group, a carboxy group, a C₁₋₆ alkyl-carbonyl group, a C₂₋₆ alkenyl-carbonyl group (e.g., crotonoyl), a C₃₋₁₀ cycloalkyl-carbonyl group (e.g., cyclobutanecarbonyl, cyclopentanecarbonyl, cyclohexanecarbonyl, cycloheptanecarbonyl), a C₃₋₁₀ cycloalkenyl-carbonyl group (e.g., 2-cyclohexenecarbonyl), a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a C₆₋₁₄ aryloxy-carbonyl group (e.g., phenyloxycarbonyl, naphthyloxycarbonyl), a C₇₋₁₆ aralkyloxy-carbonyl group (e.g., benzyloxycarbonyl, phenethyloxycarbonyl), a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl), a mono- or di-C₆₋₁₄ aryl-carbamoyl group (e.g., phenylcarbamoyl), a mono- or di-C₇₋₁₆ aralkyl-carbamoyl group, a 5- to 14-membered aromatic heterocyclylcarbamoyl group (e.g., pyridylcarbamoyl), a thiocarbamoyl group, a mono- or di-C₁₋₆ alkyl-thiocarbamoyl group (e.g., methylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a 5- to 14-membered aromatic heterocyclylthiocarbamoyl group (e.g., pyridylthiocarbamoyl), a sulfino group, a C₁₋₆ alkylsulfinyl group (e.g., methylsulfinyl, ethylsulfinyl), a sulfo group, a C₁₋₆ alkylsulfonyl group, a C₆₋₁₄ arylsulfonyl group, a phosphono group and a mono- or di-C₁₋₆ alkylphosphono group (e.g., dimethylphosphono, diethylphosphono, diisopropylphosphono, dibutylphosphono).

In the present specification, examples of the "optionally substituted amino group" include an amino group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted amino group include an amino group, a mono- or di-(optionally halogenated C₁₋₆ alkyl)amino group (e.g., methylamino, trifluoromethylamino, dimethylamino, ethylamino, diethylamino, propylamino, dibutylamino), a mono- or di-C₂₋₆ alkenylamino group (e.g., diallylamino), a mono- or di-C₃₋₁₀ cycloalkylamino group (e.g., cyclopropylamino, cyclohexylamino), a mono- or di-C₆₋₁₄ arylamino group (e.g., phenylamino), a mono- or di-C₇₋₁₆ aralkylamino group (e.g., benzylamino, dibenzylamino), a mono- or di-(optionally halogenated C₁₋₆ alkyl)-carbonylamino group (e.g., acetylamino, propionylamino), a mono- or di-C₆₋₁₄ aryl-carbonylamino group (e.g., benzoylamino), a mono- or di-C₇₋₁₆ aralkyl-carbonylamino group (e.g., benzylcarbonylamino), a mono- or di-5- to 14-membered aromatic heterocyclylcarbonylamino group (e.g., nicotinoylamino, isonicotinoylamino), a mono- or di-3- to 14-membered non-aromatic heterocyclylcarbonylamino group (e.g., piperidinylcarbonylamino), a mono- or di-C₁₋₆ alkoxy-carbonylamino group (e.g., tert-butoxycarbonylamino), a 5- to 14-membered aromatic heterocyclylamino group (e.g., pyridylamino), a carbamoylamino group, a (mono- or di-C₁₋₆ alkyl-carbamoyl)amino group (e.g., methylcarbamoylamino), a (mono- or di-C₇₋₁₆ aralkyl-carbamoyl) amino group (e.g., benzylcarbamoylamino), a C₁₋₆ alkylsulfonylamino group (e.g., methylsulfonylamino, ethylsulfonylamino), a C₆₋₁₄ arylsulfonylamino group (e.g., phenylsulfonylamino), a (C₁₋₆ alkyl) (C₁₋₆ alkyl-carbonyl) amino group (e.g., N-acetyl-N-methylamino) and a (C₁₋₆ alkyl) (C₆₋₁₄ aryl-carbonyl)amino group (e.g., N-benzoyl-N-methylamino).

In the present specification, examples of the "optionally substituted carbamoyl group" include a carbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted carbamoyl group include a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl, cyclohexylcarbamoyl), a mono- or di-C₆₋₁₄ aryl-carbamoyl group (e.g., phenylcarbamoyl), a mono- or di-C₇₋₁₆ aralkyl-carbamoyl group, a mono- or di-C₁₋₆ alkyl-carbonyl-carbamoyl group (e.g., acetylcarbamoyl, propionylcarbamoyl), a mono- or di-C₆₋₁₄ aryl-carbonyl-carbamoyl group (e.g., benzoylcarbamoyl) and a 5- to 14-membered aromatic heterocyclylcarbamoyl group (e.g., pyridylcarbamoyl).

In the present specification, examples of the "optionally substituted thiocarbamoyl group" include a thiocarbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted thiocarbamoyl group include a thiocarbamoyl group, a mono- or di-C₁₋₆ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a mono- or di-C₁₋₆ alkyl-carbonyl-thiocarbamoyl group (e.g., acetylthiocarbamoyl, propionylthiocarbamoyl), a mono- or di-C₆₋₁₄ aryl-carbonyl-thiocarbamoyl group (e.g., benzoylthiocarbamoyl) and a 5- to 14-membered aromatic heterocyclylthiocarbamoyl group (e.g., pyridylthiocarbamoyl).

In the present specification, examples of the "optionally substituted sulfamoyl group" include a sulfamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted sulfamoyl group include a sulfamoyl group, a mono- or di-C₁₋₆ alkyl-sulfamoyl group (e.g., methylsulfamoyl, ethylsulfamoyl, dimethylsulfamoyl, diethylsulfamoyl, N-ethyl-N-methylsulfamoyl), a mono- or di-C₂₋₆ alkenyl-sulfamoyl group (e.g., diallylsulfamoyl), a mono- or di-C₃₋₁₀ cycloalkyl-sulfamoyl group (e.g., cyclopropylsulfamoyl, cyclohexylsulfamoyl), a mono- or di-C₆₋₁₄ aryl-sulfamoyl group (e.g., phenylsulfamoyl), a mono- or di -C₇₋₁₆ aralkyl-sulfamoyl group (e.g., benzylsulfamoyl, phenethylsulfamoyl), a mono- or di-C₁₋₆ alkyl-carbonyl-sulfamoyl group (e.g., acetylsulfamoyl, propionylsulfamoyl), a mono- or di-C₆₋₁₄ aryl-carbonyl-sulfamoyl group (e.g., benzoylsulfamoyl) and a 5- to 14-membered aromatic heterocyclylsulfamoyl group (e.g., pyridylsulfamoyl).

In the present specification, examples of the "optionally substituted hydroxy group" include a hydroxyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxycarbonyl group, a 5- to 14-membered aromatic heterocyclic group, a carbamoyl group, a mono- or di-C₁₋₆ alkyl-carbamoyl group, a mono- or di -C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted hydroxy group include a hydroxy group, a C₁₋₆ alkoxy group, a C₂₋₆ alkenyloxy group (e.g., allyloxy, 2-butenyloxy, 2-pentenyloxy, 3-hexenyloxy), a C₃₋₁₀ cycloalkyloxy group (e.g., cyclohexyloxy), a C₆₋₁₄ aryloxy group (e.g., phenoxy, naphthyloxy), a C₇₋₁₆ aralkyloxy group (e.g., benzyloxy, phenethyloxy), a C₁₋₆ alkyl-carbonyloxy group (e.g., acetyloxy, propionyloxy, butyryloxy, isobutyryloxy, pivaloyloxy), a C₆₋₁₄ aryl-carbonyloxy group (e.g., benzoyloxy), a C₇₋₁₆ aralkyl-carbonyloxy group (e.g., benzylcarbonyloxy), a 5- to 14-membered aromatic heterocyclylcarbonyloxy group (e.g., nicotinoyloxy), a 3- to 14-membered non-aromatic heterocyclylcarbonyloxy group (e.g., piperidinylcarbonyloxy), a C₁₋₆ alkoxy-carbonyloxy group (e.g., tert-butoxycarbonyloxy), a 5- to 14-membered aromatic heterocyclyloxy group (e.g., pyridyloxy), a carbamoyloxy group, a C₁₋₆ alkyl-carbamoyloxy group (e.g., methylcarbamoyloxy), a C₇₋₁₆ aralkyl-carbamoyloxy group (e.g., benzylcarbamoyloxy), a C₁₋₆ alkylsulfonyloxy group (e.g., methylsulfonyloxy, ethylsulfonyloxy) and a C₆₋₁₄ arylsulfonyloxy group (e.g., phenylsulfonyloxy).

In the present specification, examples of the "optionally substituted sulfanyl group" include a sulfanyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group and a 5- to 14-membered aromatic heterocyclic group, each of which optionally has 1 to 3 substituents selected from substituent group A" and a halogenated sulfanyl group.

Preferable examples of the optionally substituted sulfanyl group include a sulfanyl (-SH) group, a C₁₋₆ alkylthio group, a C₂₋₆ alkenylthio group (e.g., allylthio, 2-butenylthio, 2-pentenylthio, 3-hexenylthio), a C₃₋₁₀ cycloalkylthio group (e.g., cyclohexylthio), a C₆₋₁₄ arylthio group (e.g., phenylthio, naphthylthio), a C₇₋₁₆ aralkylthio group (e.g., benzylthio, phenethylthio), a C₁₋₆ alkyl-carbonylthio group (e.g., acetylthio, propionylthio, butyrylthio, isobutyrylthio, pivaloylthio), a C₆₋₁₄ aryl-carbonylthio group (e.g., benzoylthio), a 5- to 14-membered aromatic heterocyclylthio group (e.g., pyridylthio) and a halogenated thio group (e.g., pentafluorothio).

In the present specification, examples of the "optionally substituted silyl group" include a silyl group optionally having "1 to 3 substituents selected from a C₁₋₆ alkyl group, a C₂₋₆ alkenyl group, a C₃₋₁₀ cycloalkyl group, a C₆₋₁₄ aryl group and a C₇₋₁₆ aralkyl group, each of which optionally has 1 to 3 substituents selected from substituent group A".

Preferable examples of the optionally substituted silyl group include a tri-C₁₋₆ alkylsilyl group (e.g., trimethylsilyl, tert-butyl(dimethyl)silyl).

More specifically, examples of the FGFR1 inhibitor that may be used in the present invention include PD-166866 (1-[2-amino-6-(3,5-dimethoxyphenyl)-pyrido(2,3-d)pyrimidin-7-yl]-3-tert-butylurea: (CAS No.: 192705-79-6), E-3810 (CAS No.: 1058137-23-7), PD-173074 (CAS No.: 219580-11-7), FGFR4-IN-1 (CAS No.: 1708971-72-5), FGFR-IN-1 (CAS No.: 1448169-71-8), FIIN-2 (CAS No.: 1633044-56-0), AZD4547 (CAS No.: 1035270-39-3), FIIN-3 (CAS No.: 1637735-84-2), NVP-BGJ398 (CAS No.: 1310746-10-1), NVP-BGJ398 (CAS No.: 872511-34-7), CH5183284 (CAS No.: 1265229-25-1), Derazantinib (CAS No.: 1234356-69-4), Derazantinib Racemate, Ferulic acid (CAS No.: 1135-24-6), SSR128129E (CAS No.: 848318-25-2), SSR128129E free acid (CAS No.: 848463-13-8), Erdafitinib (CAS No.: 1346242-81-6), BLU9931 (CAS No.: 1538604-68-0), PRN1371 (CAS No.: 1802929-43-6), S49076 (CAS No.: 1265965-22-7), LY2874455 (CAS No.: 1254473-64-7), Linsitinib (CAS No.: 867160-71-2), Dovitinib (CAS No.: 405169-16-6), Anlotinib (CAS No.: 1058156-90-3), Brivanib (CAS No.: 649735-46-6), Derazantinib (CAS No.: 1234356-69-4), Anlotinib Dihydrochloride (CAS No.: 1360460-82-7), ACTB-1003 (CAS No.: 939805-30-8), BLU-554 (CAS No.: 1707289-21-1), Rogaratinib (CAS No.: 1443530-05-9), BIBF 1120 esylate (CAS No.: 656247-18-6), TG 100572 Hydrochloride (CAS No.: 867331-64-4), ENMD-2076 (CAS No.: 934353-76-1), Brivanib alaninate (CAS No.: 649735-63-7), TG 100572 (CAS No.: 867334-05-2), BIBF 1120 (CAS No.: 656247-17-5), ENMD-2076 Tartrate (CAS No.: 1291074-87-7), TSU-68 (CAS No.: 252916-29-3), Ponatinib (CAS No.: 943319-70-8), Sulfatinib (CAS No.: 1308672-74-3), LY2784544 (CAS No.: 1229236-86-5), Dovitinib lactate (CAS No.: 692737-80-7), SU 5402 (CAS No.: 215543-92-3), FGF-401 (CAS No.: 1708971-55-4), Tyrosine kinase-IN-1 (CAS No.: 705946-27-6), PP58 (CAS No.: 212391-58-7), TG 100801 Hydrochloride (CAS No.: 1018069-81-2), Crenolanib (CAS No.: 670220-88-9), TG 100801 (CAS No.: 867331-82-6), Pazopanib Hydrochloride (CAS No.: 635702-64-6), Pazopanib (CAS No.: 444731-52-6), PD168393 (CAS No.: 194423-15-9), Apatinib (CAS No.: 1218779-75-9), Palbociclib isethionate (CAS No.: 827022-33-3), Foretinib (CAS No.: 849217-64-7), Lenvatinib (CAS No.: 417716-92-8), Tandutinib (CAS No.: 387867-13-2), and salts thereof (these compounds are referred to as compound group D). These compounds may each have one or more substituent(s) selected from those described above as long as the compound have FGFR1-inhibiting activity, preferably a 50% inhibitory concentration (IC₅₀) of 100 nM or lower against FGFR1.

The substructure (substituent, ring, etc.) of each of these compounds may be partially converted as long as the compound have FGFR1-inhibiting activity, preferably a 50% inhibitory concentration (IC₅₀) of 100 nM or lower against FGFR1.

In the present invention, the FGFR1 inhibitor is preferably CAS192705-79-6 (1-[2-amino-6-(3,5-dimethoxyphenyl)-pyrido(2,3-d)pyrimidin-7-yl]-3-tert-butylurea: CAS No.: 192705-79-6), E-3810 (CAS No.: 1058137-23-7), or PD173074 (CAS No.: 219580-11-7).

The FGFR1 inhibitor is not limited to the compounds described above, and an antisense oligonucleotide or siRNA against FGFR1 mRNA, an antibody binding to FGFR1, a dominant negative FGFR1 mutant, or the like can also be used as the FGFR1 inhibitor. Such an FGFR1 inhibitor is commercially available or can be synthesized according to a known method.

As used herein, "marker" means a cell antigen or a gene thereof that is specifically expressed depending on a predetermined cell type, such as "marker protein" and "marker gene". Preferably, a marker is a cell surface marker and this allows concentration, isolation, and/or detection of living cells. A marker can be a positive selection marker or a negative selection marker.

The detection of a marker protein can be conducted by an immunological assay, for example, ELISA, immunostaining, or flow cytometry using an antibody specific for the marker protein. The detection of a marker gene can be conducted by a method of amplifying and/or detecting nucleic acid known in the art, for example, RT-PCR, microarray, biochip, or the like. As used herein, "positive" for a marker protein means being detected to be positive by flow cytometry and "negative" therefor means being equal to or less than the lower detection limit in flow cytometry. Also, "positive" for a marker gene means being detected by RT-PCR and "negative" therefor means being equal to or less than the lower detection limit in RT-PCR.

As used herein, "expression" is defined as transcription and/or translation of a certain nucleotide sequence driven by an intracellular promoter.

As used herein, "cells" means a composition of cells, in other words, a cell population, unless otherwise specified. Accordingly, "cells" may include not only cells or a cell population of specific type, but also cells or cell populations of one or more other types. The proportion of cells of specific type in "cells" can be increased by enriching or purifying, or by depleting cells of one or more other types.

As used herein, "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the blastodisc, in that pluripotent cells cannot differentiate into the blastodisc and therefore, do not have the ability to form an individual.

As used herein, "multipotency" means the ability to differentiate into plural and limited numbers of linages of cells. For example, mesenchymal stem cells, hematopoietic stem cells, neural stem cells are multipotent, but not pluripotent.

As used herein, "pluripotent stem cells" refers to embryonic-stem cells (ES cells) and cells potentially having a pluripotency similar to that of ES cells, that is, the ability to differentiate into various tissues (all of the endodermal, mesodermal, and ectodermal tissues) in the living body. Examples of cells having a pluripotency similar to that of ES cells include "induced pluripotent stem cells" (that may be herein also referred to as "iPS cells"). In the present invention, preferably, pluripotent stem cells are human pluripotent stem cells.

Available "ES cells" include murine ES cells, such as various murine ES cell lines established by inGenious, RIKEN, and the like, and human ES cells, such as various human ES cell lines established by NIH, RIKEN, Kyoto University, Cellartis, and the like. For example, available ES cell lines include CHB-1 to CHB-12, RUES1, RUES2, HUES1 to HUES28 from NIH, and the like; H1 and H9 from WisCell Research; and KhES-1, KhES-2, KhES-3, KhES-4, KhES-5, SSES1, SSES2, SSES3 from RIKEN, and the like.

"Induced pluripotent stem cells" refers to cells obtained by reprograming mammalian somatic cells or undifferentiated stem cells by introducing particular factors (nuclear reprogramming factors). At present, there are various "induced pluripotent stem cells" and iPS cells established by Yamanaka, et al. by introducing the 4 factors Oct3/4, Sox2, Klf4, and c-Myc into murine fibroblasts (Takahashi K, Yamanaka S., Cell, (2006) 126: 663-676); iPS cells derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors in a virus-free way (Okita K et al. Nat. Methods 2011 May; 8(5): 409-12, Okita K et al. Stem Cells. 31 (3) 458-66) may be also used. Also, induced pluripotent stem cells established by introducing the 4 factors OCT3/4, SOX2, NANOG, and LIN28 produced by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available induced pluripotent cell lines include various iPS cell lines established by NIH, Institute of Physical and Chemical Research (RIKEN), Kyoto University and the like. For example, such human iPS cell lines include the RIKEN cell lines HiPS-RIKEN-1A, HiPS-RIKEN-2A, HiPS-RIKEN-12A, and Nips-B2 and the Kyoto University cell lines Ff-WJ-18, Ff-I01s01, Ff-I01s02, Ff-I01s04, Ff-I01s06, Ff-I14s03, Ff-I14s04, QHJI01s01, QHJI01s04, QHJI14s03, QHJI14s04, RWMH15s02, Ff-MH15s02, 253G1, 201B7, 409B2, 454E2, 606A1, 610B1, 648A1, CDI cell lines MyCell iPS Cells (21525.102.10A), MyCell iPS Cells (21526.101.10A), and the like.

As used herein, "definitive endoderm cells" means cells characterized by expression of at least one marker of SOX17, FOXA2, BMP2, CER, and CXCR4.

As used herein, "primitive gut tube cells" means cells characterized by expression of at least one marker of HNF1B and HNF4A.

As used herein, "posterior foregut cells" means cells characterized by expression of at least one marker of PDX-1, HNF6, and HLXB9.

As used herein, "pancreatic progenitor cells" means cells characterized by expression of at least one marker of PDX-1, NKX6.1, PTF-1α, GATA4, and SOX9.

As used herein, "endocrine progenitor cells" means cells characterized by expression of at least one marker of chromogranin A, NeuroD, and Ngn3, and by no expression of any marker for the pancreas-associated hormone system (such as insulin). Endocrine progenitor cells may be expressing markers of Pax-4, NKX2-2, Islet-1, PDX-1, PTF-1α, etc.

Cells at each stage of differentiation can be produced using approaches described in detail below.

### 2. Insulin-producing cells

The "insulin-producing cells" of the present invention means cells characterized by expression of insulin and obtained by inducing the differentiation of pluripotent stem cells in vitro. More specifically, the "insulin-producing cells" of the present invention are characterized by comprising: cells expressing both markers of insulin and NKX6.1 (that is, insulin-positive and NKX6.1-positive cells) at a proportion of about 30% or more; and cells expressing only insulin as a marker, not both insulin and NKX6.1 (that is, insulin-positive and NKX6.1-negative cells, hereinafter referred to as "Ins+NKX- cells"), at a proportion of more than about 15%. Herein, "insulin-positive" is also referred to as "Ins+", NKX6.1-positive as "NKX+", and NKX6.1-negative as "NKX-". "insulin-positive and NKX6.1-positive cells" are also referred to as "Ins+NKX+ cells", and "insulin-positive and NKX6.1-negative cells" as "Ins+NKX- cells".

The upper limit of the proportion of Ins+NKX+ cells in the insulin-producing cells is not particularly limited, and can be preferably about 50% or less.

The proportion of Ins+NKX- cells in the insulin-producing cells can be preferably about 20% or more, more preferably about 25% or more, further preferably about 30% or more. The upper limit of the proportion of Ins+NKX- cells in the insulin-producing cells is not particularly limited, and can be preferably about 40% or less.

For example, the insulin-producing cells comprise: Ins+NKX+ cells at a proportion of about 30% or more and about 50% or less; and Ins+NKX- cells at a proportion of more than about 15% and about 40% or less, preferably at a proportion of about 20% or more and about 40% or less, more preferably at a proportion of about 25% or more and about 40% or less, further preferably at a proportion of about 30% or more and about 40% or less.

Herein, the proportion of cells of specific type in the insulin-producing cells means the proportion to the total number of cells comprised in the insulin-producing cells. The proportion of cells of each type indicates a value in insulin-producing cells to be subjected to induction of differentiation into pancreatic islet-like cells (that is, subjected to transplantation into the living body).

Because more Ins+NKX- cells are found in immature insulin-producing cells (in an early stage of differentiation), a higher proportion of Ins+NKX- cells indicates that the insulin-producing cells are more immature insulin-producing cells (in an earlier stage of differentiation).

The insulin-producing cells can be further characterized by one or more selected from the following a to f:
a. a low expression level of a MafA gene or a protein thereof,
b. a low proportion of Ki67-positive cells,
c. a low proportion of glucagon-positive and insulin-negative cells,
d. a characteristic to exhibit glucose-stimulated insulin secretion response,
e. a high proportion of chromogranin A-positive cells, and
f. a low proportion of alkaline phosphatase-positive pluripotent stem cells.
   Here, "more" means 2, 3, 4, 5, or 6.

### a. A low expression level of a MafA gene or a gene product thereof

The insulin-producing cells of the present invention are characterized in that the expression level of a MafA gene or a protein encoded by the MafA gene is lower than the expression level of a MafA gene or a protein encoded by the MafA gene in the pancreatic islet.

"Pancreatic islet" means cells in a more advanced stage of differentiation than insulin-producing cells, including mature pancreatic β cells and characterized by expression of at least one of MafA, UCN3, and IAPP, which are markers of mature pancreatic β cells. A pancreatic islet isolated from a healthy individual can be used.

Comparison of expression levels of the MafA gene or a protein encoded by the MafA gene between insulin-producing cells and the pancreatic islet can be conducted using an approach known in the art, for example, in such a manner that the expression level of the MafA gene or a protein encoded by the MafA gene detected and quantified using an approach of, for example, RT-PCR, microarray, biochip, Western blotting, ELISA, immunostaining, or flow cytometry is corrected with the expression level of an internal standard gene or a protein encoded by the internal standard gene to obtain a relative value, which is used for comparison. "Internal standard gene" is not particularly limited, and GAPDH (glyceraldehyde-3-phosphate dehydrogenase), β-actin, β2-microglobuline, HPRT 1 (hypoxanthine phosphoribosyltransferase 1), etc., can be used.

The expression level of the MafA gene or a protein encoded by the MafA gene in the insulin-producing cells is about 20% or less, preferably about 15% or less, more preferably about 10% or less, further preferably about 5% or less, especially preferably about 1% or less of the expression level of the MafA gene or a protein encoded by the MafA gene in the pancreatic islet.

Because the MafA gene or a protein encoded by MafA gene is a marker of mature pancreatic β cells, the present feature indicates that the insulin-producing cells of the present invention comprise very few mature pancreatic β cells, or that the insulin-producing cells of the present invention are in such a stage of differentiation that the insulin-producing cells of the present invention comprise only a low proportion of mature pancreatic β cells.

### b. A low proportion of Ki67-positive cells

The insulin-producing cells of the present invention are characterized by comprising Ki67-positive cells at a proportion of less than 3%.

"Ki67-positive cells" means highly proliferative cells coexisting in insulin-producing cells formed by induction of the differentiation of pluripotent stem cells and characterized by expression of Ki67 as a marker. "Ki67" is known as a cell cycle-related nucleoprotein and is also known as a marker of cell proliferation and cell cycle because its expression is found in the G1, S, G2, and M phases of proliferating cells and is not found in the G0 phase, a quiescent stage.

Hereinafter, "Ki67-positive" is also referred to as "Ki67+". "Ki67-positive cells" is also referred to as "Ki67+ cells".

The proportion of Ki67+ cells in the insulin-producing cells is about less than 3%, about less than 1.5%, preferably about less than 1%, more preferably about less than 0.8%, further preferably about less than 0.5%.

The present feature indicates that the insulin-producing cells of the present invention comprise very few coexisting or remaining Ki67+ cells, or comprise only a low proportion of coexisting or remaining Ki67+ cells. Ki67+ cells might adversely affect recipients or influence the long-term graft survival of transplanted cells because of the high proliferative capacity, and coexisting or remaining Ki67+ cells are not preferred in some cases.

### c. A low proportion of glucagon-positive and insulin-negative cells

The insulin-producing cells of the present invention are characterized by comprising glucagon-positive and Ins- cells at a proportion of less than 3%. Hereinafter, "glucagon-positive" is also referred to as "Gcg+". "glucagon-positive and insulin-negative cells" are also referred to as "Gcg+Ins- cells".

The proportion of Gcg+Ins- cells in the insulin-producing cells is about 2.5% or less, preferably about 2% or less, more preferably about 1% or less, further preferably about 0.5% or less.

Because Gcg+Ins- is a marker of mature pancreatic α cells, the present feature indicates that the insulin-producing cells of the present invention comprise very few mature pancreatic α cells, or that the insulin-producing cells of the present invention are in such a stage of differentiation that the insulin-producing cells of the present invention comprise only a low proportion of mature pancreatic α cells.

### d. A characteristic to exhibit glucose-stimulated insulin secretion response

The insulin-producing cells of the present invention exhibit glucose-stimulated insulin secretion (GSIS) response.

The GSIS response by the insulin-producing cells can be evoked in accordance with a conventionally known approach (e.g., U.S. Patent Application No. 11/773,944), and can be evaluated, for example, by measuring the amount of C-peptide secreted into a medium. C-peptide is a decomposition product produced in an amount of moles equal to that of insulin during maturation of proinsulin. Measurement of the amount of C-peptide can be performed, for example, through ELISA using an anti-C-peptide monoclonal antibody.

### e. A high proportion of chromogranin A-positive cells

The insulin-producing cells of the present invention are characterized by comprising chromogranin A-positive cells at a proportion of more than about 45%.

Hereinafter, "chromogranin A-positive" is also referred to as "Chga+". "Chromogranin A-positive cells" is also referred to as "Chga+ cells".

The proportion of Chga+ cells in the insulin-producing cells can be preferably about 50% or more (e.g., about 55% or more), more preferably about 60% or more, more preferably about 70% or more, more preferably about 80% or more, more preferably about 85% or more, especially preferably about 90% or more. The upper limit of the proportion of Chga+ cells in the insulin-producing cells is not particularly limited, and can be, for example less than about 95%, or less than about 99%. The proportion of Chga+ cells in the insulin-producing cells may be about 93% or more.

Chga+ cells include cells that secrete a hormone such as insulin (endocrine cells), which also include the above Ins+NKX+ cells and Ins+NKX- cells. Accordingly, the present feature indicates that the insulin-producing cells of the present invention comprise endocrine cells at a high proportion.

### f. A low proportion of alkaline phosphatase-positive pluripotent stem cells

The insulin-producing cells of the present invention are characterized by comprising alkaline phosphatase-positive pluripotent stem cells at a proportion of less than about 0.01%.

The proportion of alkaline phosphatase-positive pluripotent stem cells in the insulin-producing cells can be preferably about 0.008% or less, more preferably about 0.005% or less, further preferably about 0.001% or less.

Because alkaline phosphatase is a marker indicative of an undifferentiated state of pluripotent stem cells, the present feature indicates that the insulin-producing cells of the present invention comprise very few unintended pluripotent stem cells that have not undergone induction of differentiation, or comprise unintended pluripotent stem cells that have not undergone induction of differentiation at a low proportion.

Alkaline phosphatase-positive pluripotent stem cells may be further expressing an additional marker indicative of pluripotency. For the additional marker indicative of the pluripotency of pluripotent stem cells, at least one selected from Nanog, Sox2, SSEA-1, SSEA-3, SSEA-4, TRA-1-60, TRA-1-81, etc., can be used.

The insulin-producing cells of the present invention may be in a cryopreserved state. The insulin-producing cells of the present invention under cryopreservation can differentiate into pancreatic islet-like cells and mature to secrete a hormone after thawing, as with the case of fresh insulin-producing cells that have not been cryopreserved. Cryopreservation and thawing can be performed using an approach commonly used in the art.

The insulin-producing cells of the present invention can be produced with approaches described in detail below.

### 3. Pancreatic islet-like cells

As used herein, "pancreatic islet-like cells" means mature cells obtained by inducing the differentiation of the above-described insulin-producing cells, and, as with the case of "pancreatic islet" given by pancreas development in the living body, characterized by expressing at least one marker of MafA, UCN3, and IAPP, which are maturation markers of pancreatic β cells, and expressing glucagon, which is a maturation marker of pancreatic α cells.

More specifically, "pancreatic islet-like cells" herein can be characterized by one or more selected from the following (a) to (d):
(a) a high proportion of chromogranin A-positive cells (Chga+ cells),
(b) a low proportion of Ki67-positive cells (Ki67+ cells),
(c) a high proportion of glucagon-positive and insulin-negative cells (Gcg+Ins- cells), and
(d) a characteristic to exhibit insulin-secreting action in response to hypoglycemia.
Here, "more" means 2, 3, or 4.

Evaluation can be conducted on the presence or absence of the features (a) to (d) 1 week, preferably 2 weeks after induction of the differentiation of the insulin-producing cells (e.g., after transplantation into the living body), where the upper limit of the period is not particularly limited, and can be within 1 year. The proportion of cells of specific type in pancreatic islet-like cells means the proportion of cell clusters derived from a graft to the total number of cells.

### (a) A high proportion of Chga+ cells

The pancreatic islet-like cells of the present invention are characterized by comprising Chga+ cells at a proportion of about 50% or more. The proportion of Chga+ cells in the pancreatic islet-like cells is preferably about 60% or more, more preferably about 70% or more, furthermore preferably about 90% or more, especially preferably about 95% or more (e.g., about 97% or more, about 98% or more).

Chga+ cells include cells that secrete a hormone such as insulin and glucagon (endocrine cells), and the present feature indicates that the pancreatic islet-like cells comprise endocrine cells at a high proportion.

### (b) A low proportion of Ki67+ cells

The pancreatic islet-like cells of the present invention are characterized by comprising Ki67-positive cells at a proportion of less than about 3%. The proportion of Ki67-positive cells in the pancreatic islet-like cells is preferably less than about 1%, more preferably less than about 0.8%, further preferably less than about 0.5%.

The present feature indicates that the pancreatic islet-like cells comprise very few Ki67+ cells, coexistence or remains of which may be unpreferred, or comprise Ki67+ cells at a very low proportion.

### (c) A high proportion of Gcg+Ins- cells

The pancreatic islet-like cells of the present invention are characterized by comprising Gcg+Ins- cells at a proportion of about 10% or more. The proportion of Gcg+Ins- cells in the pancreatic islet-like cells is preferably about 15% or more, more preferably about 20% or more, furthermore preferably about 25% or more. The upper limit of the proportion of Gcg+Ins- cells in the pancreatic islet-like cells is not particularly limited, and can be, for example, about 50% or less, preferably about 45% or less, more preferably about 40% or less.

Because being a marker of mature pancreatic α cells, Gcg+Ins- indicates that the pancreatic islet-like cells comprise mature pancreatic α cells at a higher proportion than insulin-producing cells. Gcg+Ins- indicates that the pancreatic islet-like cells are in a more mature stage of differentiation than insulin-producing cells.

### (d) A characteristic to exhibit insulin-secreting action in response to hypoglycemia

The pancreatic islet-like cells of the present invention are characterized by exhibiting insulin-secreting action in response to hypoglycemia. "Insulin-secreting action in response to hypoglycemia" means that the amount of secreted insulin increases within 1 hour, 2 hours, 3 hours, 4 hours, or 5 hours after the occurrence of hypoglycemia. "Hypoglycemia" means the case that the glucose concentration in blood or in a medium is about 70 mg/dL or less. Measurement of the amount of secreted insulin can be performed using any conventionally known approach, which is not particularly limited, and can be achieved by measuring the amount of C-peptide in blood or in a medium.

On the occurrence of hypoglycemia in the living body, in normal cases, the elevation of the blood glucose level is promoted through secretion of glucagon or the like, and at the same time insulin is secreted as antagonism against glucagon, controlling the blood glucose level. Similarly, the pancreatic islet-like cells of the present invention enable control of blood glucose levels against hypoglycemia, and exhibit an action of promoting the elevation of the blood glucose level in response to hypoglycemia and at the same time secreting insulin as antagonism against the elevation of the blood glucose level.

### 4. Method for producing cells

The insulin-producing cells of the present invention can be obtained by inducing the differentiation of pluripotent stem cells. Induction of the differentiation of pluripotent stem cells into insulin-producing cells can be performed using the following steps of induction of differentiation:
step 1) inducing the differentiation of pluripotent stem cells into definitive endoderm cells;
step 2) inducing the differentiation of the definitive endoderm cells into primitive gut tube cells;
step 3) inducing the differentiation of the primitive gut tube cells into posterior foregut cells;
step 4) inducing the differentiation of the posterior foregut cells into pancreatic progenitor cells;
step 5) inducing the differentiation of the pancreatic progenitor cells into endocrine progenitor cells; and
step 6) inducing the differentiation of the endocrine progenitor cells into insulin-producing cells.

Hereinafter, each step will be described, though the induction of differentiation into each cell is not limited by these approaches.

### Step 1) Differentiation into definitive endoderm cells

The pluripotent stem cells are cultured in a medium containing a low dose of activin A to be allowed to differentiate into definitive endoderm cells.

The medium used in this step may be a basal medium for use in the culture of mammalian cells, such as RPMI medium, MEM medium, iMEM medium, DMEM (Dulbecco's Modified Eagle Medium) medium, Improved MEM Zinc Option medium, Improved MEM/1% B-27 supplement/Penicillin Streptomycin medium, or MCDB131/20 mM Glucose/NaHCO₃/FAF-BSA/ITS-X/Glutamax/ascorbic acid/Penicillin Streptomycin medium.

Activin A can be contained in the medium at a low dose, for example, 5 to 100 ng/mL, preferably 5 to 50 ng/mL, more preferably 5 to 10 ng/mL.

The medium can be further supplemented with a ROCK inhibitor and a GSK3β inhibitor.

The concentration of the GSK3β inhibitor in the medium is appropriately set depending on the type of the GSK3β inhibitor used. For example, in the case of using CHIR as the GSK3β inhibitor, its concentration is usually 2 to 5 µM, preferably 2 to 4 µM, particularly preferably about 3 µM.

The concentration of the ROCK inhibitor in the medium is appropriately set depending on the type of the ROCK inhibitor used. For example, in the case of using Y27632 as the ROCK inhibitor, its concentration is usually 5 to 20 µM, preferably 5 to 15 µM, particularly preferably about 10 µM.

The medium can be further supplemented with insulin. The insulin can be contained in an amount of 0.01 to 20 µM, preferably 0.1 to 10 µM, more preferably 0.5 to 5 µM, in the medium. The concentration of the insulin in the medium may be, but is not limited to, the concentration of insulin contained in added B-27 supplement.

The number of cells at the start of culture is not particularly limited and is 22000 to 150000 cells/cm², preferably 22000 to 100000 cells/cm², more preferably 22000 to 80000 cells/cm². The culture period is 1 day to 4 days, preferably 1 day to 3 days, particularly preferably 3 days.

The culture temperature is not particularly limited, and the culture is performed at 30 to 40°C (for example, 37°C). The concentration of carbon dioxide in a culture container is on the order of, for example, 5%.

Alternatively, in the present invention, the pluripotent stem cells can be subjected to first culture in a medium under conditions that allow the action of insulin in the presence of a low dose of activin A and subsequently subjected to second culture in a medium under conditions that do not allow the action of insulin for production of definitive endoderm cells.

### (1) First culture

"Conditions that allow the action of insulin" means conditions that cause the activation of the insulin signal transduction pathway in cells by insulin. In normal cases, insulin binds to the insulin receptor present on cell membrane surfaces to activate tyrosine kinase present within the receptor, thereby tyrosine-phosphorylating the insulin receptor substrate protein family (IRS: IRS-1, 2, 3). Herein, the occurrence of the series of reactions that is initiated by binding of insulin to the insulin receptor is expressed as "cause the activation of the insulin signal transduction pathway".

Examples of the conditions that allow the action of insulin include the case that insulin is contained in a medium. Insulin can be of any type that can activate the insulin signal transduction pathway in the pluripotent stem cells, and may be insulin produced using a recombinant method or insulin produced through synthesis using a solid-phase synthesis method. For example, insulin derived from a human, a nonhuman primate, a pig, cattle, a horse, sheep, a goat, a llama, a dog, a cat, a rabbit, a mouse, or a guinea pig can be used, and human insulin is preferred.

In the present invention, any insulin mutant, insulin derivative, or insulin agonist that can cause the activation of the insulin signal transduction pathway in the pluripotent stem cells can be used as "insulin". Examples of "insulin mutant" include: an insulin mutant possessing a polypeptide consisting of an amino acid sequence formed by deletion, substitution, addition, or insertion of 1 to 20 amino acids, preferably 1 to 10 amino acids, further preferably 1 to 5 amino acids, in the amino acid sequence of insulin and being capable of causing the activation of the insulin signal transduction pathway; and an insulin mutant possessing a polypeptide consisting of an amino acid sequence having a sequence identity of 80% or higher, more preferably 90% or higher, further preferably 95% or higher, the most preferably 99% or higher, to the amino acid sequence of insulin, and being capable of causing the activation of the insulin signal transduction pathway. Comparison of amino acid sequences can be performed using a known approach, and, for example, using BLAST (Basic Local Alignment Search Tool at the National Center for Biological Information), for example, with default settings. "Insulin derivative" means: a polypeptide consisting of an amino acid sequence formed by chemical substitution (e.g., α-methylation, α-hydroxylation), deletion (e.g., deamination), or modification (e.g., N-methylation) of some groups in the amino acid residues of insulin or an insulin mutant and being capable of causing the activation of the insulin signal transduction pathway; or a substance that exhibits the same action. "Insulin agonist" means a polypeptide capable of causing the activation of the insulin signal transduction pathway by binding to the insulin receptor irrespectively of the structure of insulin, or a substance that exhibits the same action.

Insulin can be contained in an amount of 0.01 to 20 µM, preferably 0.1 to 10 µM, more preferably 0.5 to 5 µM, in the medium for the first culture. The concentration of the insulin in the medium may be, but is not limited to, the concentration of insulin contained in added B-27 supplement.

The medium can be further supplemented with a ROCK inhibitor and/or a GSK3β inhibitor. The concentration of the ROCK inhibitor in the medium is appropriately set depending on the type of the ROCK inhibitor used. For example, in the case of using Y27632 as the ROCK inhibitor, its concentration is usually 5 to 20 µM, and can be preferably 5 to 15 µM, particularly preferably about 10 µM. The concentration of the GSK3β inhibitor in the medium is appropriately set depending on the type of the GSK3β inhibitor used. For example, in the case of using CHIR as the GSK3β inhibitor, its concentration is usually 2 to 5 µM, and can be preferably 2 to 4 µM, particularly preferably about 3 µM.

The medium can be further supplemented with one or more selected from the group consisting of a pyruvate (e.g., a sodium salt), L-alanyl L-glutamine, and glucose. The medium can be supplemented with a pyruvate in an amount of 10 to 1000 mg/L, preferably 30 to 500 mg/L, more preferably 50 to 200 mg/L, particularly preferably about 110 mg/L. The medium can be supplemented with L-alanyl L-glutamine in an amount of 50 to 2000 mg/L, preferably 100 to 1500 mg/L, more preferably 500 to 1000 mg/L, particularly preferably about 860 mg/L. The medium can be supplemented with glucose in an amount of 15 mM or more, preferably 15 to 30 mM, more preferably 15 to 25, particularly preferably about 25 mM. The concentrations of the pyruvate, L-alanyl L-glutamine, and glucose in the medium may be, but are not limited to, the concentrations of the pyruvate, L-alanyl L-glutamine, and glucose contained in the DMEM medium (DMEM, high glucose, GlutaMAX (TM), pyruvate (Thermo Fisher Scientific)) or other DMEM medium.

A medium prepared with any of the above basal media as a base supplemented with one or more of the above components can be used for the medium. The basal medium is preferably DMEM medium, more preferably DMEM medium containing a pyruvate, L-alanyl L-glutamine, and glucose in the above amounts.

The culture period of the first culture can be in a range selected from 6 hours to 48 hours, preferably 12 to 24 hours. The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture. For two-dimensional culture, the number of cells at the start of culture is not particularly limited and can be 50000 to 1000000 cells/cm², preferably 150000 to 300000 cells/cm², more preferably about 200000 cells/cm². For three-dimensional culture, the number of cells at the start of culture is not particularly limited and can be 10000 to 1000000 cells/mL, preferably 100000 to 500000 cells/mL.

### (2) Second culture

"Conditions that do not allow the action of insulin" means conditions that do not cause the activation of the insulin signal transduction pathway in cells by insulin. "Do not cause the activation of the insulin signal transduction pathway" not only means causing completely no activation of the insulin signal transduction pathway, but also means causing only a slight activation of the insulin signal transduction pathway to such a degree that any significant difference as compared with the activation of the insulin signal transduction pathway in the absence of insulin is not found. Thus, examples of "conditions that do not allow the action of insulin" include the case that no insulin is contained in a medium, or, even if insulin is contained in a medium, the amount is such that only a slight activation is caused to such a degree that the significant difference is not found. Alternatively, "conditions that do not allow the action of insulin" means that even if insulin is contained in a medium, the activation of the insulin signal transduction pathway is not caused by virtue of inclusion of an insulin signal inhibitor together. "Insulin signal inhibitor" means a component capable of blocking the insulin signal transduction pathway at any stage. Examples of the insulin signal inhibitor include polypeptides and compounds that bind to or compete with any of insulin, the insulin receptor, various proteins that act as a signaling substance, etc., to inhibit the intermolecular interaction in which such factors are involved. Examples of such an insulin signal inhibitor include LY294002 [2-(4-morpholinyl)-8-phenyl-4H-1-benzopyran-4-one], which competes with and inhibits binding of ATP to the catalytic subunit of PI3 kinase. The insulin signal inhibitor is not limited to these, and antibodies that bind to any of insulin, the insulin receptor, and various proteins that act as a signaling substance or dominant-negative mutants of the antibodies, and antisense oligonucleotides, siRNA, and the like for mRNA for any of the insulin receptor and various proteins that act as a signaling substance can also be used as the insulin signal inhibitor. The insulin signal inhibitor is commercially available or can be synthesized according to a known method.

The medium can be further supplemented with a ROCK inhibitor and/or a GSK3β inhibitor. The amount(s) of the ROCK inhibitor and/or GSK3β inhibitor in the medium can be selected from the ranges described above in the first culture, and may be the same as or different from the amount(s) for use in the first culture.

The medium can be further supplemented with one or more selected from the group consisting of a pyruvate, L-alanyl L-glutamine, and glucose. The amounts of the pyruvate, L-alanyl L-glutamine, and glucose in the medium can be selected from the ranges described above in the first culture, and may be the same as or different from the amounts for use in the first culture.

A medium prepared with a basal media for use in the culture of mammalian cells as a base supplemented with one or more of the above components can be used for the medium for use in the second culture. For the basal medium, the media described above in the first culture can be used, and the basal medium may be the same as or different from the basal medium for use in the first culture. The basal medium is preferably DMEM medium, more preferably DMEM medium containing a pyruvate, L-alanyl L-glutamine, and glucose in the above amounts.

The culture period of the second culture is at least 6 hours, and can be in a range selected preferably from 6 hours to 72 hours, further preferably from 24 to 72 hours. The culture temperature is not particularly limited, and the culture is performed at 30 to 40°C (for example, 37°C). The culture may be performed by any of two-dimensional culture and three-dimensional culture. The concentration of carbon dioxide in a culture container is on the order of, for example, 5%.

The media in the first culture and the second culture can be supplemented with the above low dose of activin A. The amount of activin A contained in the medium in the first culture and the amount of activin A contained in the medium in the second culture may be the same or different.

The media in the first culture and the second culture may be further supplemented with dimethyl sulfoxide.

The proportion of endocrine cells obtained after step 6) can be increased by culturing the pluripotent stem cells in the presence of a low dose of activin A, or by subjecting the pluripotent stem cells to the first culture in a medium under conditions that allow the action of insulin in the presence of a low dose of activin A and subsequently to the second culture in a medium under conditions that do not allow the action of insulin.

### Step 2) Differentiation into primitive gut tube cells

The definitive endoderm cells obtained in step 1) are further cultured in a medium containing a growth factor to induce their differentiation into primitive gut tube cells. The culture period is 2 days to 8 days, preferably about 4 days.

The culture temperature is not particularly limited, and the culture is performed at 30 to 40°C (for example, 37°C). The concentration of carbon dioxide in a culture container is on the order of, for example, 5%.

Any of the basal media for use in the culture of mammalian cells described above in Step 1) can be used as culture medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

### Step 3) Differentiation into posterior foregut cells

The primitive gut tube cells obtained in step 2) are further cultured in a medium containing a growth factor, cyclopamine, noggin, and the like to induce their differentiation into posterior foregut cells. The culture period is 1 day to 5 days, preferably about 2 days.

The culture temperature is not particularly limited, and the culture is performed at 30 to 40°C (for example, 37°C). The concentration of carbon dioxide in a culture container is on the order of, for example, 5%.

Any of the basal media for use in the culture of mammalian cells described above in Step 1) can be used as culture medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

The concentration of the cyclopamine in the medium is not particularly limited and is usually 0.5 to 1.5 µM, preferably 0.3 to 1.0 µM, particularly preferably about 0.5 µM.

The concentration of the noggin in the medium is not particularly limited and is usually 10 to 200 ng/mL, preferably 50 to 150 ng/mL, particularly preferably about 100 ng/mL.

The medium may also be supplemented with dimethyl sulfoxide.

### Step 4) Differentiation into pancreatic progenitor cells

The posterior foregut cells obtained in step 3) may be further cultured in a medium containing a factor having CDK8/19-inhibiting activity, preferably a medium containing a factor having CDK8/19-inhibiting activity and a growth factor, to induce their differentiation into pancreatic progenitor cells. The culture period is 2 days to 10 days, preferably about 5 days.

According to the previous report (Toyoda et al., Stem cell Research (2015) 14, 185-197), the posterior foregut cells obtained in step 3) are treated and dispersed by pipetting with 0.25% trypsin-EDTA and the dispersion is subjected to centrifugal separation to obtain cell suspension and then the suspension is reseeded to a fresh medium of step 4).

As in step 1), a basal medium for use in the culture of mammalian cells can be used as culture medium. The medium may be appropriately supplemented with a serum replacement, a vitamin, an antibiotic, and the like, in addition to the growth factor.

Each of the compounds mentioned above or salts thereof can be used as the factor having CDK8/19-inhibiting activity. The amount of the factor added to the medium is appropriately determined according to the compound or the salt thereof used and is usually about 0.00001 µM to 5 µM, preferably 0.00001 µM to 1 µM. The concentration of the factor having CDK8/19-inhibiting activity in the medium is preferably a concentration that attains inhibitory activity of 50% or more for CDK8/19.

The growth factor is preferably EGF, KGF, and/or FGF10, more preferably KGF and/or EGF, further preferably KGF and EGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF and EGF as the growth factor, the concentration of KGF is usually 5 to 150 ng/mL, preferably 30 to 100 ng/mL, particularly preferably about 50 ng/mL, and the concentration of EGF is usually 10 to 200 ng/mL, preferably 50 to 150 ng/mL, particularly preferably about 100 ng/mL.

Culture on the first day in step 4) may be performed in the presence of a ROCK inhibitor, and culture on the following days may be performed in a medium containing no ROCK inhibitor.

The medium may also contain a PKC activator. PdBU (PKC activator II), TPB (PKC activator V), or the like is used as the PKC activator, though the PKC activator is not limited thereto. The concentration of activin A to be added is about 0.1 to 100 ng/ml, preferably about 1 to 50 ng/ml, more preferably about 3 to 10 ng/ml.

The medium may also be supplemented with dimethyl sulfoxide.

In any of the steps, the medium may be supplemented with a serum replacement (for example, B-27 supplement, ITS-G), in addition to the components described above. Also, an amino acid, L-glutamine, GlutaMAX (product name), a non-essential amino acid, a vitamin, an antibiotic (for example, Antibiotic-Antimycotic (also referred to as AA herein), penicillin, streptomycin, or a mixture thereof), an antimicrobial agent (for example, amphotericin B), an antioxidant, pyruvic acid, a buffer, inorganic salts, and the like may be added thereto, if necessary. In the case of adding an antibiotic to the medium, its concentration in the medium is usually 0.01 to 20% by weight, preferably 0.1 to 10% by weight.

The cell culture is performed by adherent culture without the use of feeder cells. For the culture, a culture container, for example, a dish, a flask, a microplate, or a cell culture sheet such as OptiCell (product name) (Nunc), is used. The culture container is preferably surface-treated in order to improve adhesiveness to cells (hydrophilicity), or coated with a substrate for cell adhesion such as collagen, gelatin, poly-L-lysine, poly-D-lysine, laminin, fibronectin, Matrigel (for example, BD Matrigel (Nippon Becton Dickinson Company, Ltd.)), or vitronectin. The culture container is preferably a culture container coated with type I-collagen, Matrigel, fibronectin, vitronectin or poly-D-lysine, more preferably a culture container coated with Matrigel or poly-D-lysine.

The culture temperature is not particularly limited, and the culture is performed at 30 to 40°C (for example, 37°C). The concentration of carbon dioxide in a culture container is on the order of, for example, 5%.

### Step 5) Differentiation into endocrine progenitor cells

The pancreatic progenitor cells obtained in step 4) are further cultured in a medium containing a growth factor to induce their differentiation into endocrine progenitor cells. The culture period is 2 days to 3 days, preferably about 2 days.

Any of the basal media for use in the culture of mammalian cells described above in Step 1) can be used as culture medium. The medium is supplemented with SANT1, retinoic acid, ALK5 inhibitor II, T3, and LDN according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. The medium may also be supplemented with dimethyl sulfoxide.

The cell culture is performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

### Step 6) Differentiation into insulin-producing cells

The endocrine progenitor cells obtained in step 5) are further cultured in a medium containing an FGFR1 inhibitor to induce their differentiation into insulin-producing cells. The culture period is 14 days to 30 days, preferably about 14 to 20 days.

Any of the basal media for use in the culture of mammalian cells described above in Step 1) can be used as culture medium. The medium is supplemented with ALK5 inhibitor II, T3, LDN, γ-secretase inhibitor XX, γ-secretase inhibitor RO, N-cysteine, an AXL inhibitor, and ascorbic acid according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. For example, the medium may be supplemented with ALK5 inhibitor II, T3, LDN, γ-secretase inhibitor RO, and ascorbic acid or may be supplemented with T3, ALK5 inhibitor II, ZnSO₄, heparin, N-acetylcysteine, Trolox, and R428.

The cell culture is performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

The FGFR1 inhibitor can be contained in any amount capable of inhibiting FGFR1 activity in the medium, and can be contained in an amount of, for example, 10 µM or less or 5 µM or less, preferably in an amount of less than 5 µM, less than 4 µM, less than 3 µM, or less than 2 µM. The lower limit of the amount of the FGFR1 inhibitor to be added is not particularly limited and can be 0.1 µM or more, preferably 0.5 µM or more. The amount of the FGFR1 inhibitor to be added is preferably less than 5 µM and 0.1 µM or more, more preferably less than 5 µM and 0.5 µM or more. The culture in the presence of the FGFR1 inhibitor can be performed for at least 12 hours, preferably 24 hours or longer, 2 days or longer, 4 days or longer, 8 days or longer, 10 days or longer, or 15 days or longer. The culture in the presence of the FGFR1 inhibitor is preferably performed for 4 days or longer. The culture in the presence of the FGFR1 inhibitor can be performed, for example, for about last 4 to 15 days, preferably about last 4 to 7 days, of step 6). The medium may be replaced during the period of treatment with the FGFR1 inhibitor and can be replaced with a medium supplemented with the FGFR1 inhibitor, having the same or different composition as or from that before the replacement, according to the culture schedule.

Culturing cells in a medium containing the FGFR1 inhibitor can inhibit the proliferation of Ki67-positive cells in the insulin-producing cells to be obtained.

The insulin-producing cells obtained in step 6) can be cryopreserved until use.

### 5. Differentiation into pancreatic islet-like cells

The insulin-producing cells of the present invention can be induced to differentiate into pancreatic islet-like cells by transplanting into a living body of an animal.

"Animal" is preferably a mammal. Examples thereof include humans, nonhuman primates, pigs, cattle, horses, sheep, goats, llamas, dogs, cats, rabbits, mice, and guinea pigs. A human is preferred.

The transplantation is preferably performed to an *in vivo* region where the cells can be fixed at a given position, and can be performed, for example, subcutaneously, intraperitoneally, to the peritoneal mesothelium, to the greater omentum, to a fat tissue, to a muscle tissue, or beneath the capsule of each organ such as the pancreas or the kidney, in the animal. The number of cells to be transplanted may vary depending on factors such as the age and body weight of a recipient, and the size of a transplantation site and is not particularly limited. For example, the number of cells can be on the order of 10 × 10⁴ cells to 10 × 10¹¹ cells. The transplanted cells are induced to differentiate in an *in vivo* environment and can thereby differentiate into pancreatic islet-like cells. The transplanted insulin-producing cells differentiate and mature into pancreatic islet-like cells 1 week, preferably 2 weeks after the transplantation, though the period may vary depending on factors such as the number of cells to be transplanted, the age and body weight of a recipient, and the transplantation site. The pancreatic islet-like cells obtained may then be recovered or may be indwelled *in vivo* as they are.

### 6. Applications

The insulin-producing cells of the present invention can be safely administered as they are or in the form of a medicament containing the cells mixed with a pharmacologically acceptable carrier, etc., to a patient in need thereof.

"Pharmacologically acceptable carrier" may be any pharmacologically acceptable carrier that allows transplantation together with the insulin-producing cells into the living body and allows the transplanted insulin-producing cells to differentiate and mature into pancreatic islet-like cells, and any pharmacologically acceptable carrier according to a desired dosage form can be appropriately used. Examples of "pharmacologically acceptable carrier" include a hydrogel. The hydrogel is preferably a hydrogel consisting of biocompatible and/or biodegradable polymer, and a hydrogel consisting of one or more selected from alginic acid, fibronectin, gelatin, collagen, proteoglycan, glucosaminoglycan (e.g., hyaluronic acid, chondroitin sulfate, dermatan sulfate, heparan sulfate, heparin, keratan sulfate), laminin, and vitronectin, and salts or esters of them can be used as such a hydrogel. For the hydrogel, for example, a gel containing alginic acid prepared using a salt of alginic acid (e.g., sodium alginate, calcium alginate, ammonium alginate) or an ester of alginic acid (also referred to as propylene glycol alginate) (WO2010/032242 and WO2011/154941) can be suitably used. The hydrogel can be used with the insulin-producing cells dispersed in the gel.

The insulin-producing cells or medicament can be contained in a device capable of accommodating the insulin-producing cells or medicament. The device may be any device that allows transplantation together with the insulin-producing cells or medicament into the living body and allows the transplanted insulin-producing cells to differentiate and mature into pancreatic islet-like cells, and a device of any shape and material can be used. For such a device, for example, a device consisting of biocompatible and/or biodegradable polymer (e.g., polyglycolic acid, polylactic acid), ceramic, or metal (e.g., titanium, stainless steel, cobalt, or any alloy of them) can be used. The shape of the device is not particularly limited, and can be the shape of a capsule, a bag, a chamber, or the like that can accommodate the insulin-producing cells or medicament. The device preferably has a porous part, a hole, a path, or the like that allows the inside and the outside to communicate with each other.

The insulin-producing cells or medicament of the present invention can be supplied in a cryopreserved form, and can be used by thawing in use.

The insulin-producing cells or medicament of the present invention can be used by transplanting into the living body of a patient in need thereof. The transplantation is preferably performed to an *in vivo* region where the cells can be fixed at a given position, and can be performed, for example, subcutaneously, intraperitoneally, to the peritoneal mesothelium, to the greater omentum, to a fat tissue, to a muscle tissue, or beneath the capsule of each organ such as the pancreas or the kidney. Preferred is subcutaneous transplantation, which is less invasive. The insulin-producing cells to be transplanted can be suitably administered in a therapeutically effective amount, which may vary depending on factors such as the age and body weight of a recipient, the size of a transplantation site, and the severity of a disease and is not particularly limited. For example, the therapeutically effective amount can be on the order of 10 × 10⁴ cells to 10 × 10¹¹ cells.

The transplanted insulin-producing cells differentiate and mature in the living body to become pancreatic islet-like cells, and exert functions useful for treatment or prophylaxis of diseases, disorders, or symptoms. That is, the insulin-producing cells or medicament of the present invention can be used as a prodrug.

With the insulin-producing cells or medicament of the present invention, pancreatic islet-like cells can be formed in the living body of a patient, and the blood glucose level in a patient can be improved and/or retained by the action of insulin and glucagon secreted by pancreatic islet-like cells.

Therefore, the insulin-producing cells or medicament of the present invention can be used for treatment or prophylaxis of a disease, a disorder, or a symptom for which improvement and/or retention of blood glucose levels are/is needed. Examples of the disease, disorder, or symptom include, but are not limited to, diabetes mellitus, abnormal fasting and postprandial glucose levels, and hypoglycemia in a patient, in particular, a patient with diabetes mellitus (e.g., hypoglycemia by administration of insulin in a patient with diabetes mellitus). "Treatment" means treatment, curing, prevention, or amelioration of remission of a disease, a disorder, or a symptom, or reduction of the progression speed of a disease, a disorder, or a symptom. "Prophylaxis" means reduction of the possibility or risk of the onset of a disease, a disorder, or a symptom, or retardation of the onset of a disease, a disorder, or a symptom.

The patient is a mammal, (for example, a mouse, a rat, a hamster, a rabbit, a cat, a dog, cattle, sheep, a monkey, and a human), preferably a human.

Hereinafter, the present invention will be described with reference to Examples. However, the present invention is not limited by these Examples.

### Examples

### Example 1: Degree of maturation of insulin-producing cells and proportion of coexisting proliferative cells 1. Method

### (1) Preparation of insulin-producing cells

The induction of differentiation of the iPS cell line Ff-I14s04 into insulin-producing cells was carried out according to step 1) to 6) above or the previous report (Nature Biotechnology 2014; 32: 1121-1133).

Specifically, the iPS cells were suspended in an iPS cell medium containing 10 µM ROCK inhibitor (Y-27632), counted with the cell counter NC200 (ChemoMetec), adjusted to 37.5 × 10⁴ cells/mL, and seeded for incubation on a plate coated with iMatrix at 2 mL/well (75 × 10⁴ cells/well). Then, first culture was performed under conditions that allow the action of insulin, that is, in a medium (DMEM-high glucose-GlutaMAX-pyruvate/2% B27 (with insulin)/Penicillin Streptomycin/1% dimethyl sulfoxide) containing a differentiation-inducing factor (GSK3β inhibitor (3 µM CHIR99021), low dose (10 ng/mL) of activin A), and subsequently second culture was performed under conditions that do not allow the action of insulin, that is, in a medium (DMEM-high glucose-GlutaMAX-pyruvate/2% B27 (without insulin)/Penicillin Streptomycin/1% dimethyl sulfoxide) containing a differentiation-inducing factor (low dose (10 ng/mL) of activin A) to obtain definitive endoderm cells. The definitive endoderm cells obtained were cultured in a medium (Improved MEM/1% B27 (with insulin)/Penicillin Streptomycin) containing 50 ng/mL KGF, and subsequently in a medium (Improved MEM/1% B27 (with insulin)/Penicillin Streptomycin) containing 50 ng/mL KGF, 100 ng/mL noggin, 0.5 µM cyclopamine, 10 nM TTNPB, and 250 µM vitamin C, and the resulting posterior foregut cells were recovered. The posterior foregut cells were suspended in a medium (Improved MEM/1% B27 (with insulin)/Penicillin Streptomycin) containing 0.1 µM ROCK inhibitor, 100 ng/mL KGF, 50 ng/mL EGF, 10 mM nicotinamide, and 250 µM vitamin C, adjusted to 175 × 10⁴ cells/mL, and reseeded for culture on a plate coated with iMatrix at 350 × 10⁴ cells/well. The resulting cells were recovered again, suspended in a medium (Improved MEM/1% B27 (with insulin)/Penicillin Streptomycin) containing 0.25 µM SANT-1, 50 nM retinoic acid, 10 µM ALK5 inhibitor II, 100 nM LDN, 1 µM T3, 50 ng/mL bFGF, 1 µM XAV, and 10 µM Y-27632, adjusted to 20 × 10⁴ cells/mL, and reseeded for culture on a nonadherent 96-well plate (Sumitomo Bakelite Co., Ltd.) at 3 × 10⁴ cells/well.

The resulting cell population was cultured in a medium (Improved MEM/1% B27 (with insulin)/Penicillin Streptomycin) containing 10 µM ALK5 inhibitor II, 1 µM T3, 100 µM LDN, 1 µM γ-secretase inhibitor (RO-4929097), 250 µM ascorbic acid, and 1 µM FGF receptor 1 inhibitor (PD-166866) to obtain insulin-producing cells.

### (2) Evaluation of protein expressions

Protein expressions (insulin (INS), NKX6.1, glucagon (GCG), Ki67) in the insulin-producing cells prepared from the iPS cell line Ff-I14s04 and a human pancreatic islet isolated from a healthy individual (hereinafter, referred to as the "islet") were measured by flow cytometry. As a control, the prototype of insulin-producing cells prepared without treatment with the FGF receptor 1 inhibitor (PD-166866) (hereinafter, referred to as the "prototype") was subjected to measurement in the same manner.

### (3) Evaluation of gene expression levels

With cDNAs synthesized from total RNA fractions collected from samples of the prototype, insulin-producing cells, and islet, mRNA expression levels of insulin, glucagon, and MafA and UCN3, which are known as markers of mature pancreatic β cells, were measured with a quantitative PCR method. Normalization was carried out with the expression level of GAPDH mRNA, as a control gene, measured in the same manner.

### 2. Results

### (1) Evaluation of protein expressions

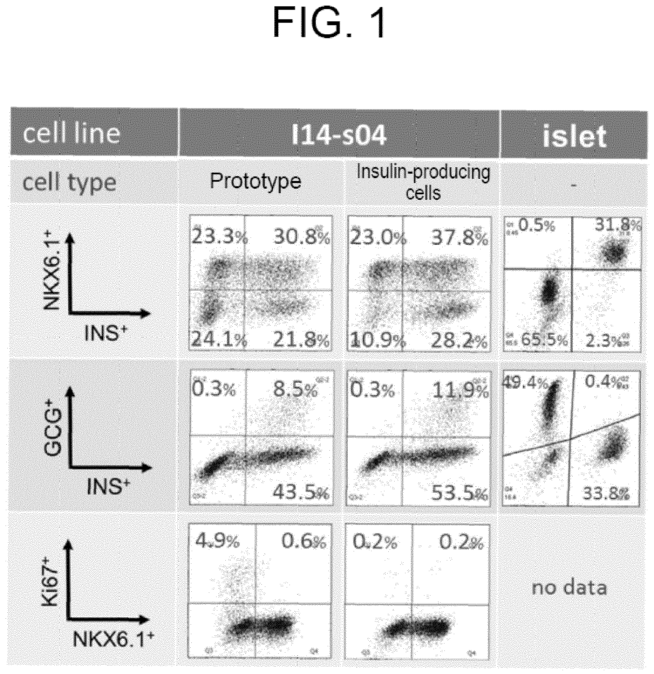

Figure 1 shows results of flow cytometry measurement. Table 1 shows proportions of insulin-positive/NKX6.1-positive cells, proportions of insulin-positive/NKX6.1-negative cells, proportions of chromogranin A-positive cells, and proportions of Ki67-positive cells. The insulin-producing cells exhibited a proportion of insulin-positive/NKX6.1-positive cells comparable to or higher than that in the islet. On the other hand, the proportion of insulin-positive/NKX6.1-negative cells was found to be clearly higher than that in the islet. Comparison between the insulin-producing cells and the prototype found that the insulin-producing cells exhibited increased proportions of insulin-positive/NKX6.1-positive cells, insulin-positive/NKX6.1-negative cells, and chromogranin A-positive cells, and exhibited a markedly decreased proportion of Ki67-positive cells.

**[Table 1]**

| Cell line | Ff-I14-s04 | | |
|---|---|---|---|
| Cell type | Prototype | Insulin-producing cells | islet |
| Proportion of insulin-positive/NKX6.1-positive cells | 30.8 % | 37.8 % | 31.8 % |
| Proportion of chromogranin A-positive cells | 83.9 % | 94.7 % | 88.7 % |
| Proportion of insulin-positive/N KX6.1 -negative cells | 21.8 % | 28.2 % | 2.3 % |
| Proportion of Ki67-positive cells | 5.5 % | 0.4 % | n.d. |

| | | | |
|---|---|---|---|
| n.d., not determined | | | |

### (2) Evaluation of gene expression levels

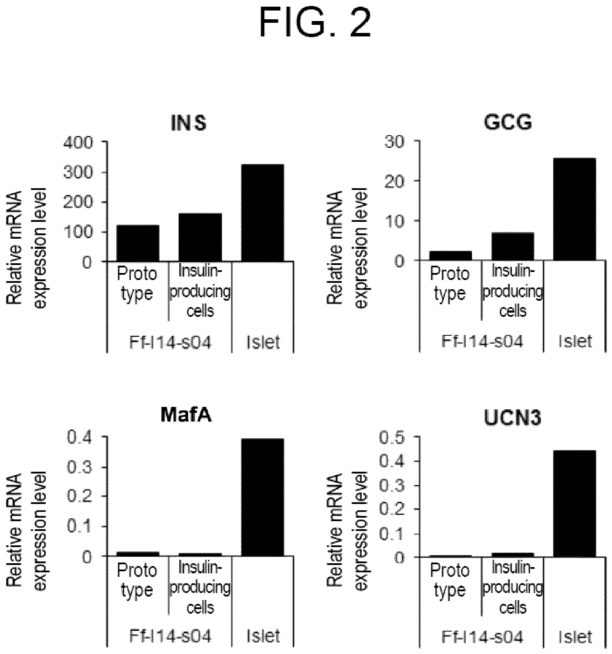

Table 2 and Figure 2 show expression levels of insulin, glucagon, MafA, and UCN3 in the prototype, insulin-producing cells, and islet.

In accord with the above results on proportions of insulin-positive cells and proportions of glucagon-positive cells by flow cytometry, the insulin-producing cells were found to exhibit increased expression levels of insulin and glucagon as compared with those in the prototype, and the expression levels reached ∼50% of those in the islet. By contrast, the expression levels of MafA and UCN3 in the insulin-producing cells were revealed to be extremely lower than those in the human pancreatic islet.

**[Table 2]**

| Cell line | Ff-I14-s04 | | |
|---|---|---|---|
| Cell type | Prototype | Insulin-producing cells | islet |
| INS gene | 121 | 162 | 326 |
| GCG gene | 2.4 | 6.9 | 25.7 |
| MafA gene | 0.01 | 0.01 | 0.39 |
| UCN3 gene | 0.01 | 0.02 | 0.44 |

The above results indicate that the insulin-producing cells are a cell aggregate including β cells in a proportion comparable to that in the pancreatic islet but are not a completely mature pancreatic islet yet. In addition, the insulin-producing cells were confirmed to include extremely few proliferative unintended cells (Ki67-positive cells).

### Example 2: Proportion of coexisting iPS cells retaining undifferentiated state in insulin-producing cells 1. Method

### High-sensitivity detection of iPS cells retaining undifferentiated state (High efficient culture cell assay)

Definitive endoderm cells obtained by culturing the iPS cells Ff-I14s04 in a medium (RPMI/1% B27 (with insulin)/Penicillin Streptomycin/dimethyl sulfoxide) containing a differentiation-inducing factor (GSK3β inhibitor, ROCK inhibitor, low dose of activin A) were induced to differentiate into pancreatic progenitor cells (intermediate cells in the course of generation of insulin-producing cells). A suspension of 6 × 10⁵ cells of the pancreatic progenitor cells was spiked with 0, 6 (0.001%), or 30 (0.005%) cells of iPS cells retaining an undifferentiated state, and the resultant was seeded on a 10-cm dish and cultured under conditions of AK03N medium and iMatrix-511 coating, which are conditions preferred for iPS cells regaining an undifferentiated state, for 7 days. Under the same conditions, single 6 × 10⁵ cells of the pancreatic progenitor cells without spiking with the iPS cells were cultured. After the completion of the culture, generated colonies of iPS cells retaining an undifferentiated state were visualized by staining with alkaline phosphatase, and the number was counted.

### 2. Results

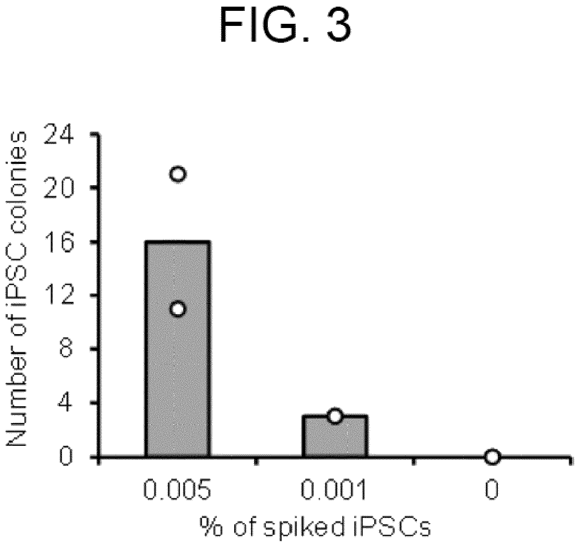

Figure 3 shows the results. In the culture dishes for pancreatic progenitor cells spiked with 6 cells and 30 cells of the iPS cells retaining an undifferentiated state, 3 and 16 colonies were observed on average, respectively. In the culture dish for pancreatic progenitor cells without spiking, by contrast, completely no colonies were observed. These results indicated that the proportion of coexisting iPS cells retaining an undifferentiated state is 0.001% or less in pancreatic progenitor cells generated by the current method of inducing differentiation and insulin-producing cells to be generated thereafter.

The above results confirmed that the insulin-producing cells include no or extremely few coexisting iPS cells retaining an undifferentiated state.

### Example 3: Influence of freezing and thawing on insulin-producing cells

### 1. Method

### (1) Reaggregation of insulin-producing cells after freezing and thawing

Insulin-producing cells prepared with the same method as the method described in Example 1 except that definitive endoderm cells were obtained by culturing the iPS cells Ff-I14s04 in a medium (RPMI/1% B27 (with insulin)/Penicillin Streptomycin/dimethyl sulfoxide) containing a differentiation-inducing factor (GSK3β inhibitor, ROCK inhibitor, low dose of activin A) were frozen with a slow freezing method using a commercially available cryopreservation solution (CryoStor CS10 (BioLifeSolutions Inc.)). Specifically, 3 × 10⁶ cells were suspended in 1 mL of the cryopreservation solution, and injected into a freezing vial. The vial was placed in a freezing vessel (BICELL, Nihon Freezer Co., Ltd.), and preserved at -80°C.

In thawing, the freezing vial was warmed with a ThawSTAR Cell Thawing System (Astero Bio Corporation) for quick thawing. The thawed cell suspension was added to 10 mL of culture solution. After the supernatant was removed through centrifugal separation, the cells were suspended in a medium containing 10 µM ROCK inhibitor (Y-27632), and the viable cell count and cell survival rate after thawing were measured using the cell counter NC200 (ChemoMetec). The cells after thawing were seeded in a porous plate (Kuraray Co., Ltd.), and subjected to three-dimensional culture to prepare a cell aggregate.

### (2) Transplantation of insulin-producing cells after freezing and thawing into living body

The insulin-producing cells after freezing and thawing were transplanted under kidney capsule using immunodeficient NOD/SCID mice having streptozotocin-induced insulin-deficient diabetes mellitus. For follow-up after the transplantation, human C-peptide concentrations in blood and blood glucose levels were measured.

### 2. Results

### (1) Reaggregation of insulin-producing cells after freezing and thawing

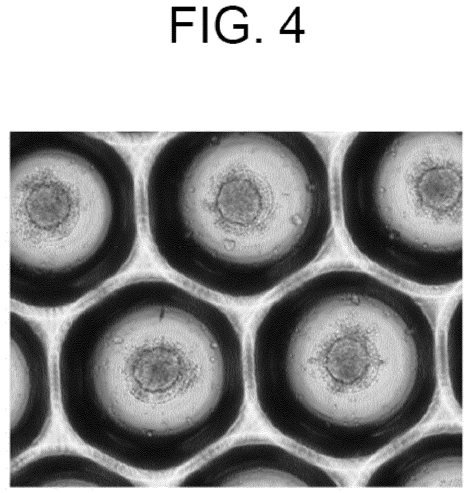

The cell survival rate immediately after thawing was 80.9%. Of the cells injected into the freezing vial, 77.0% were successfully recovered as viable cells. Figure 4 shows a phase-contrast microscopic image of cells cultured by three-dimensional culture for 4 days. As shown in Figure 4, it is clear that the cryopreserved cells survive and retain the ability to form a cell aggregate.

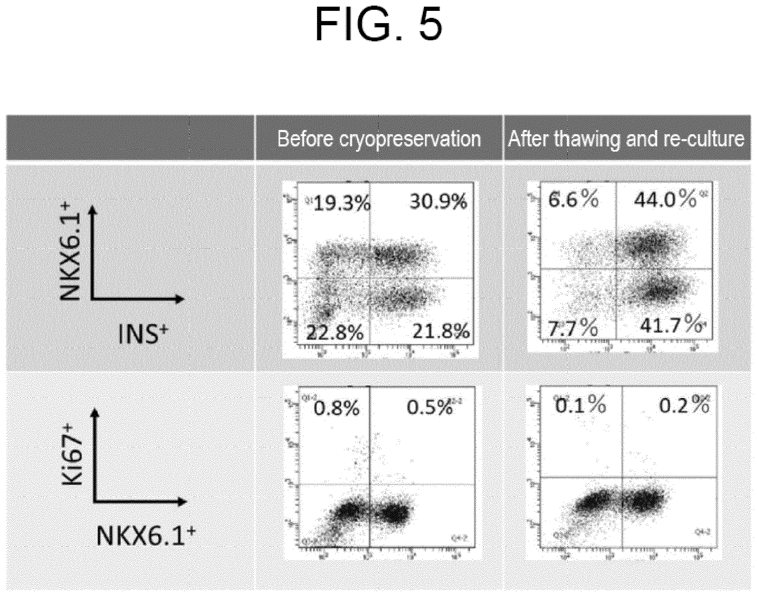

Figure 5 shows results of flow cytometry measurement of expressions of INS, NKX6.1, and Ki67 in cells before cryopreservation and after thawing and re-culture. Table 3 shows insulin-positive rates and proportions of Ki67-positive cells. As shown in Figure 5 and Table 3, there were no decrease of the proportion of insulin-positive cells and no increase of the number of Ki67-positive cells after cryopreservation.

**[Table 3]**

| | Before cryopreservation | After thawing and re-culture |
|---|---|---|
| Proportion of insulin-positive cells | 52.7 % | 85.7 % |
| Proportion of Ki67-positive cells | 1.3 % | 0.3 % |

### (2) Transplantation of insulin-producing cells into living body

Results about human C-peptide concentrations in blood and blood glucose levels measured for follow-up after transplantation are shown in Table 4. The insulin-producing cells transplanted under kidney capsule after freezing and thawing were found to secrete human C-peptide into blood and exhibited an effect of improving high blood glucose, 4 months after transplantation.

**[Table 4]**

| | At transplantation | 4 months after transplantation |
|---|---|---|
| Human C-peptide concentration in blood (pM) | n.d. | 982.4 (N=1) |
| Blood glucose level (mg/dL) | 500 (N=1) | 117 (N=1) |

These results confirmed that the insulin-producing cells retain the efficiency of induction of differentiation even after freezing and thawing.

### Example 4: Insulin-producing cells transplanted into living body

### 1. Method

### (1) Transplantation of insulin-producing cells into living body

Insulin-producing cells prepared with the same method as in Example 3 were transplanted under kidney capsule using immunodeficient NOD/SCID mice having streptozotocin (STZ)-induced diabetes mellitus, and subcutaneously transplanted using NOD/SCID mice having Akita gene mutation that causes the spontaneous onset of diabetes mellitus. In the subcutaneous transplantation, Fibrin gel was used as a carrier for the insulin-producing cells. After transplantation, human C-peptide (an index of insulin derived from the insulin-producing cells) concentrations in blood and blood glucose levels were measured to evaluate graft survival of the insulin-producing cells.

### (2) Evaluation of response of insulin-producing cells to glucose loading or hypoglycemia

To mice having transplanted insulin-producing cells that had achieved graft survival, glucose was orally administered forcedly to temporarily elevate blood glucose levels, or the insulin formulation glargine was subcutaneously administered to induce hypoglycemia, and response of the insulin-producing cells thereafter was evaluated by measuring human C-peptide concentrations in blood and glucagon concentrations in blood. As a control, non-diabetes mellitus NOD/SCID mice without transplantation were used, and blood concentrations of endogenous pancreatic islet-derived mouse C-peptide and glucagon were measured.

### 2. Results

### (1) Evaluation of graft survival of insulin-producing cells transplanted into living body

Table 5 shows results of measurement of human C-peptide concentrations in blood and blood glucose levels after transplantation. For any animal and transplantation site, human C-peptide was detected in blood 3 to 4 months after transplantation. In the mice, high blood glucose was improved in 3 mounts after transplantation, and blood glucose levels retained at normal levels for 5 to 6 months, indicating long-term graft survival of the transplanted insulin-producing cells.

**[Table 5]**

| Animal-transplantation site | Human C-peptide concentration in blood (pM) | | Blood glucose level (mg/dL) | | |
|---|---|---|---|---|---|
| | 3 to 4 months after transplantation | After 5 to 6 months | At transplantation | After 3 months | After 5 to 6 months |
| STZ NOD/SCID Mice-under kidney capsule | 898±335 (N=3) | 915, 1060 (N=2) | 516±33 (N=4) | 113±64 (N=3) | 117,91 (N=2) |
| Akita NOD/SCID Mice-subcutaneous | 2436±516 (N=4) | 2195±882 (N=4) | 562±31 (N=4) | 83±27 (N=4) | 61±7 (N=3) |

| | | | | | |
|---|---|---|---|---|---|
| Mean ± standard deviation for N = 3 or more, individual data for N = 2 | | | | | |

### (2) Evaluation of response of insulin-producing cells to glucose loading or hypoglycemia

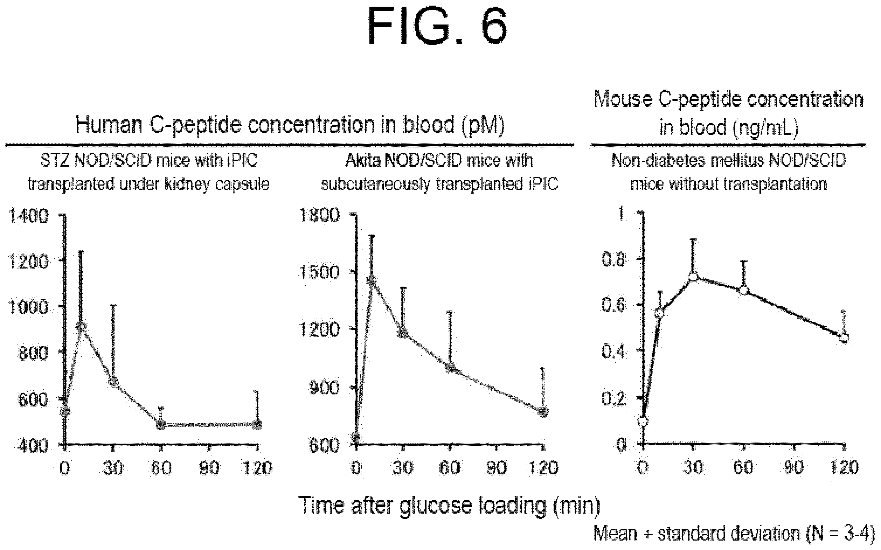

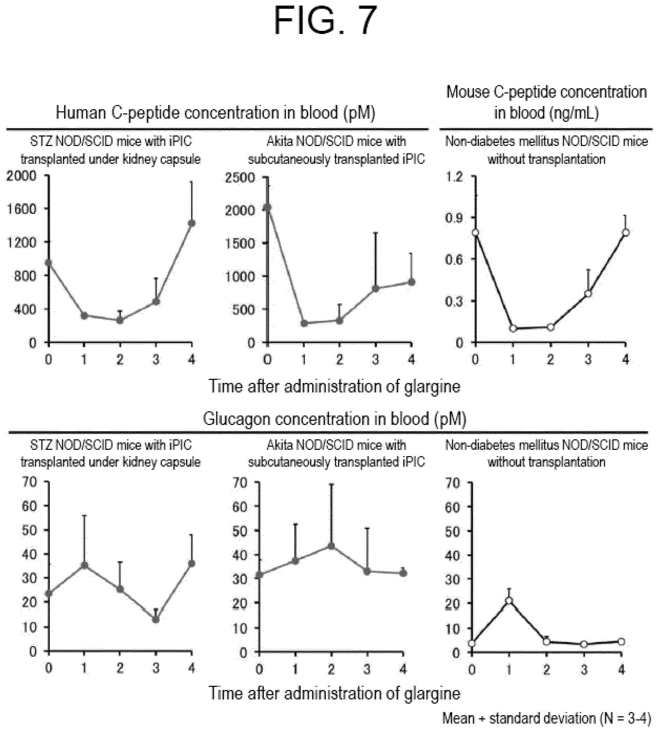

Figure 6 shows human C-peptide concentrations in blood after glucose loading, and Figure 7 shows human C-peptide concentrations in blood and glucagon concentrations in blood after administration of glargine. After 3 to 4.5 months after transplantation of the insulin-producing cells, human C-peptide concentrations in blood temporarily increased as a result of glucose loading, and, on the other hand, decreased when hypoglycemia was induced by administration of glargine. These changes were similar to the changes in the blood concentration of endogenous mouse C-peptide in the non-diabetes mellitus mice without transplantation. The mice with the transplanted insulin-producing cells exhibited higher glucagon concentrations in blood than the non-diabetes mellitus mice without transplantation, which suggested that the insulin-producing cells were releasing glucagon into blood.

The above results indicated that the insulin-producing cells transplanted in the living body achieve long-term graft survival and exert physiological insulin-regulating action similar to that of the endogenous pancreatic islet in response to the variation of the blood glucose level.

### Example 5: Subcutaneously transplanted insulin-producing cells

### 1. Method

### (1) Transplantation of insulin-producing cells into living body

Insulin-producing cells dispersed in an alginate hydrogel were subcutaneously transplanted into immunodeficient NOD/SCID mice having streptozotocin-induced insulin-deficient diabetes mellitus. For follow-up after the transplantation, human C-peptide concentrations in blood and blood glucose levels were measured. Grafts were excised 3 months after transplantation.

### (2) Evaluation of protein expressions in grafts

Each excised graft was treated to separate into single cells, which were fixed and subjected to flow cytometry measurement of protein expressions (insulin, NKX6.1, glucagon, chromogranin A) in the insulin-producing cells. In addition, the insulin-producing cells in the excised grafts and those at transplantation were fixed and dehydrated, from which frozen sections were prepared to evaluate expressions of intended proteins (insulin, glucagon) by immunohistological staining.

### 2. Results

### (1) Transplantation of insulin-producing cells into living body

Results about human C-peptide concentrations in blood and blood glucose levels measured for follow-up after transplantation are shown in Table 6. The subcutaneously transplanted insulin-producing cells were found to secrete human C-peptide into blood and exhibited an effect of improving high blood glucose, 1 to 3 months after transplantation.

**[Table 6]**

| | At transplantation | 1 month after transplantation | 3 months after transplantation |
|---|---|---|---|
| Human C-peptide concentration in blood (pM) | n.d. | 490.4, 307.6 (N=2) | 714.3 (N=1) |
| Blood glucose level (mg/dL) | 568, 457 (N=2) | 238, 196 (N=2) | 62 (N=1) |

### (2) Evaluation of protein expressions in grafts

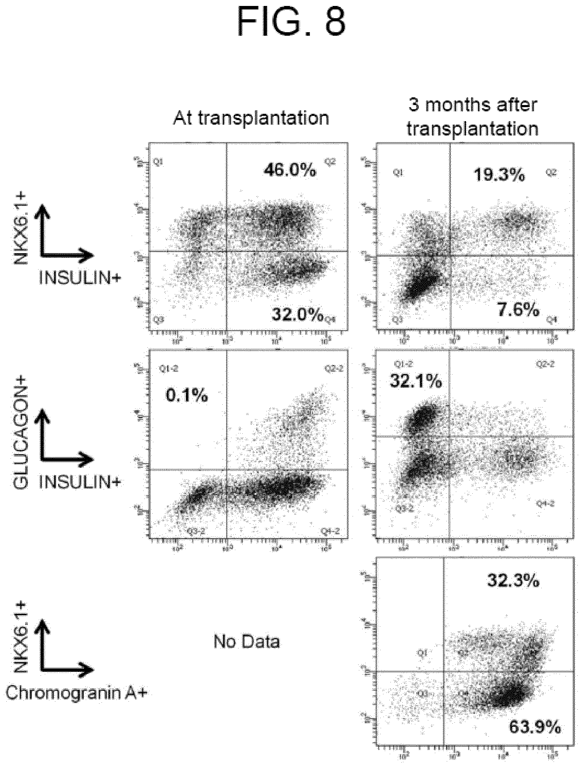

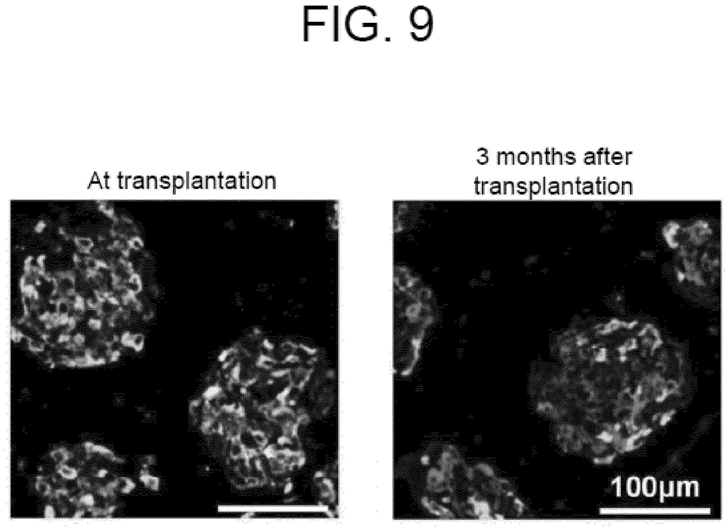

Table 7 and Figure 8 show results of measurement of proportions of insulin-positive/NKX6.1-positive cells, proportions of insulin-positive/NKX6.1-negative cells, proportions of glucagon-positive/insulin-negative cells, and chromogranin A-positive rates. For the graft 3 months after transplantation, an increased proportion of glucagon-positive/insulin-negative cells and a decreased proportion of insulin-positive/NKX6.1-negative cells were found as compared with those at transplantation. In addition, the graft 3 months after transplantation was found to be composed of a high proportion of chromogranin A-positive endocrine cells. Results of immunohistological staining for an excised graft are shown in Figure 9. For the graft 3 months after transplantation, an increased proportion of cells positive only for glucagon was found as compared with that at transplantation, suggesting achievement of differentiation into mature pancreatic islet-like cells in the living body.

**[Table 7]**

| | At transplantation | 3 months after transplantation |
|---|---|---|
| Proportion of insulin-positive/NKX6.1-positive cells | 46.0 % | 19.3 % |
| Proportion of insulin-positive/NKX6.1-negative cells | 32.0 % | 7.6 % |
| Proportion of glucagon-positive/insulin-negative cells | 0.1% | 32.1 % |
| Proportion of chromogranin A-positive cells | n.d. | 96.2 % |

The above results confirmed that the insulin-producing cells subcutaneously transplanted into diabetes mellitus mice retain high proportions of endocrine cells and differentiate into mature pancreatic islet-like cells including mature α cells in the living body.

In addition, it was confirmed that pancreatic islet-like cells that have matured in the living body include extremely few (e.g., less than 3%) coexisting proliferative unintended cells (Ki67-positive cells).

## Claims

1. Insulin-producing cells comprising: insulin-positive and NKX6.1-positive cells at a proportion of 30% or more; and insulin-positive and NKX6.1-negative cells at a proportion of more than 15%.

2. The insulin-producing cells according to claim 1, wherein an expression level of a MafA gene or a gene product thereof is lower than an expression level of a MafA gene or a gene product thereof in a pancreatic islet.

3. The insulin-producing cells according to claim 1 or 2, comprising Ki67-positive cells at a proportion of less than 3%.

4. The insulin-producing cells according to any one of claims 1 to 3, comprising glucagon-positive and insulin-negative cells at a proportion of less than 3%.

5. The insulin-producing cells according to any one of claims 1 to 4, wherein the insulin-producing cells exhibit glucose-stimulated insulin secretion (GSIS) response.

6. The insulin-producing cells according to any one of claims 1 to 5, comprising chromogranin A-positive cells at a proportion of more than 45%.

7. The insulin-producing cells according to any one of claims 1 to 6, comprising alkaline phosphatase-positive pluripotent stem cells at a proportion of less than 0.01%.

8. A medicament comprising the insulin-producing cells according to any one of claims 1 to 7.

9. The medicament according to claim 8, wherein the insulin-producing cells are accommodated in a device.

10. The medicament according to claim 8 or 9, wherein the insulin-producing cells are dispersed in a hydrogel.

11. The medicament according to any one of claims 8 to 10, to be used for transplantation into a living body.

12. The medicament according to any one of claims 8 to 11, to be used for subcutaneous transplantation.

13. The medicament according to any one of claims 8 to 12, for use in treatment of diabetes mellitus.

14. The medicament according to any one of claims 8 to 12, for use in improvement and/or retention of control of fasting and postprandial glucose levels in a patient.

15. The medicament according to any one of claims 8 to 12, to be used to reduce risk of hypoglycemia in a patient with diabetes mellitus.

16. The medicament according to any one of claims 8 to 12, for use in a method for inducing differentiation into pancreatic islet-like cells in a living body.

17. The medicament according to claim 16, wherein pancreatic islet-like cells differentiated in a living body comprise chromogranin A-positive cells at a proportion of 50% or more.

18. The medicament according to claim 16 or 17, wherein pancreatic islet-like cells differentiated in a living body comprise Ki67-positive cells at a proportion of less than 3%.

19. The medicament according to any one of claims 16 to 18, wherein pancreatic islet-like cells differentiated in a living body comprise glucagon-positive and insulin-negative cells at a proportion of 10% or more.

20. The medicament according to any one of claims 16 to 19, wherein pancreatic islet-like cells differentiated in a living body exhibit insulin-secreting action in response to hypoglycemia.

21. A method for producing insulin-producing cells comprising: insulin-positive and NKX6.1-positive cells at a proportion of 30% or more; and insulin-positive and NKX6.1-negative cells at a proportion of more than 15%, the method comprising the steps of:
culturing pluripotent stem cells in the presence of a low dose of activin A to produce definitive endoderm cells; and
culturing endocrine progenitor cells in a medium containing an FGFR1 inhibitor to produce the insulin-producing cells.

22. A method for generating pancreatic islet-like cells, the method comprising
transplanting insulin-producing cells into a living body to induce differentiation into pancreatic islet-like cells, wherein the insulin-producing cells comprise:
insulin-positive and NKX6.1-positive cells in a proportion of 30% or more; and insulin-positive and
NKX6.1-negative cells in a proportion of more than 15%.

23. A method for treating diabetes mellitus, the method comprising
transplanting insulin-producing cells into a living body to induce differentiation into pancreatic islet-like cells, wherein the insulin-producing cells comprise:
insulin-positive and NKX6.1-positive cells in a proportion of 30% or more; and insulin-positive and
NKX6.1-negative cells in a proportion of more than 15%.

24. A method for improving and/or retaining control of fasting and postprandial glucose levels in a patient, the method comprising
transplanting insulin-producing cells into a living body to induce differentiation into pancreatic islet-like cells, wherein the insulin-producing cells comprise:
insulin-positive and NKX6.1-positive cells in a proportion of 30% or more; and insulin-positive and
NKX6.1-negative cells in a proportion of more than 15%.

25. A method for reducing risk of hypoglycemia in a patient with diabetes mellitus, the method comprising
transplanting insulin-producing cells into a living body to induce differentiation into pancreatic islet-like cells, wherein the insulin-producing cells comprise:
insulin-positive and NKX6.1-positive cells in a proportion of 30% or more; and insulin-positive and
NKX6.1-negative cells in a proportion of more than 15%.
